(19) Europäisches Patentamt
European Patent Office
Office européen des brevets

(11) EP 3 957 389 A1

(12) **EUROPÄISCHE PATENTANMELDUNG**

(43) Veröffentlichungstag:
**23.02.2022 Patentblatt 2022/08**

(21) Anmeldenummer: **20191588.1**

(22) Anmeldetag: **18.08.2020**

(51) Internationale Patentklassifikation (IPC):
***B01F 5/06*** (2006.01) ***A61K 8/02*** (2006.01)
***A61K 8/11*** (2006.01) ***A61K 8/73*** (2006.01)
***A61Q 19/00*** (2006.01)

(52) Gemeinsame Patentklassifikation (CPC):
**A61Q 19/00; A61K 8/0241; A61K 8/11; A61K 8/73; B01F 25/42;** A61K 2800/10; A61K 2800/41; A61K 2800/80; B01F 2025/9171; B01F 2025/91911; B01F 2101/21

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Beiersdorf AG**
**20253 Hamburg (DE)**

(72) Erfinder:
- **BURR, Franziska**
**22765 Hamburg (DE)**
- **HEROLD, Marc**
**22769 Hamburg (DE)**
- **SCHMID, Nathali**
**21075 Hamburg (DE)**

(74) Vertreter: **Haseltine Lake Kempner LLP**
**Bürkleinstrasse 10**
**80538 München (DE)**

(54) **MISCHVERFAHREN FÜR KOSMETISCHE FESTSTOFFE**

(57) Die vorliegende Erfindung betrifft ein Mischverfahren, welches die folgenden Schritte aufweist: a) Bereitstellen eines kosmetischen Feststoffs (F), b) Bereitstellen eines Fluids (S), c) Zuführen des kosmetischen Feststoffs (F) und des Fluids (S) in einen statischen Mischer (M), und d) Erhalten einer Mischung enthaltend den kosmetischen Feststoff (F) und das Fluid (S) aus dem statischen Mischer (M), sowie ein Produkt erhältlich aus diesem Verfahren. Die vorliegende Erfindung betrifft ferner die Verwendung eines statischen Mischers zum Mischen eines kosmetischen Feststoffs (F) und eines Fluids (S).

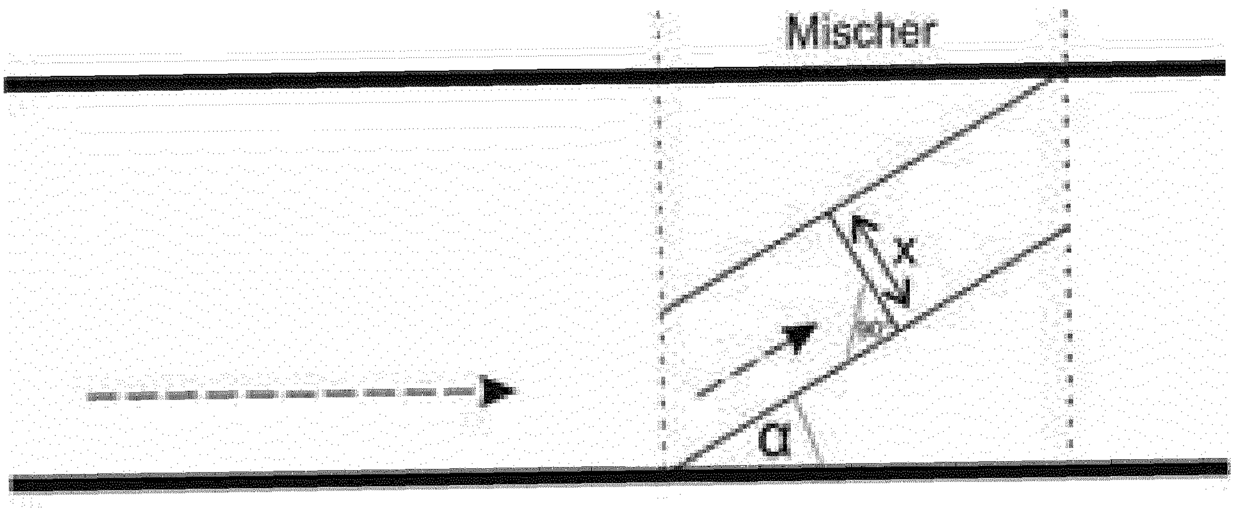


**Fig. 1**

EP 3 957 389 A1

**Beschreibung**

GEBIET DER ERFINDUNG

Die Erfindung betrifft ein Mischverfahren für kosmetische Feststoffe, insbesondere Mikrokapseln, ein Produkt erhältlich aus diesem Verfahren und die Verwendung eines statischen Mischers zum Mischen eines kosmetischen Feststoffs und eines Fluids.

STAND DER TECHNIK

In kosmetischen Produkten ist es manchmal wünschenswert, dass es erst bei der Anwendung des Produktes durch den Endverbraucher zu einer Durchmischung aller Komponenten kommt. Beispielsweise kann es bei vollständiger Durchmischung aller Komponenten bereits bei der Herstellung des Produktes zu Reaktionen der Komponenten untereinander kommen, was zu einer verkürzten Lagerstabilität führen kann. Eine vollständige Durchmischung kann auch zu einem trüben oder anderweitig optisch nicht ansprechenden Produkt führen ohne die Wirksamkeit zu beinträchtigen. Jedoch ist ein optisch ansprechenderes Produkt u. A. aus Marketinggründen zu bevorzugen. Beispielsweise kann das kosmetische Packmittel zwei Kammern aufweisen, welche verschiedene Fluide, beispielsweise Gele, beinhalten, die erst bei Anwendung gemischt werden, z.B. mittels eines entsprechenden Pumpspenders. Allerdings muss gewährleistet sein, dass eine vollständige Durchmischung stattfindet und ein festgelegtes Mischungsverhältnis eingehalten wird. Die entsprechenden Dosiereinrichtungen müssen sich folglich im kosmetischen Packmittel selbst befinden, wodurch dieses aufwändiger und teurer wird. Alternativ könnte das kosmetische Produkt aus zwei flüssigen, nicht miteinander mischbaren Phasen bestehen, die jedoch vor jeder Anwendung vermischt werden müssen, beispielsweise durch Schütteln. Jedoch wird, je nachdem wie stark geschüttelt wird, nicht die erforderliche Durchmischung erreicht. Zudem wird das komplette Produkt gemischt, so dass etwaige Reaktionen der einzelnen Bestandteile während des Entmischungsvorgangs nach der Anwendung erfolgen können. Zudem ist diese Methode für hochviskose Fluids ungeeignet.

Eine Alternative ist die Verwendung eines Fluids, in dem sich ein kosmetischer Feststoff befindet, welcher bei Entnahme aus dem kosmetischen Packmittel mit dem Fluid vermischt wird. Beispielsweise kann der Feststoff flüssige Inhaltsstoffe enthalten, zum Beispiel in Form von Mikrokapseln oder ähnlichem. Dadurch wird der kosmetische Feststoff in dem Fluid gelöst und/oder suspendiert, beispielsweise wird der kosmetische Feststoff bei Entnahme durch die Entnahmevorrichtung oder von dem Anwender zerdrückt oder ähnliches. Hierzu muss der Feststoff jedoch weich genug sein um mit geringem Kraftaufwand zerdrückt werden zu können. Allerdings muss der Feststoff hart genug sein um bei einem Mischvorgang mit weiteren Komponenten nicht beschädigt oder gar zerstört zu werden, da sonst eine unerwünschte frühzeitige Vermischung stattfindet. Die erforderliche Härte der kosmetischen Feststoffe für übliche kosmetische Mischverfahren (mechanische, dynamische Rührer) damit diese Feststoffe nicht zerstört werden, ist jedoch normalerweise so hoch, dass eine Mischung durch Zerstörung des Feststoffes bei Entnahme aus dem kosmetischen Packmittel nicht oder nur sehr eingeschränkt möglich ist.

Eine Möglichkeit die Härte des Feststoffes im nachhinein zu senken sind Feststoffe die beispielsweise aus Pektinen und/oder Alginaten hergestellt sind. Diese Komponenten bilden mit zwei- oder mehrwertigen Kationen, beispielsweise Calcium, Komplexe, wodurch die Härte dieser Komponenten erhöht wird. Dieser Vorgang ist reversibel. D.h. im nachhinein lassen sich die zwei- oder mehr Kationen durch Komplexbildner wie beispielsweise Citrat oder EDTA entfernen, wodurch die Härte der Feststoffe wieder gesenkt wird. Folglich lassen sich prinzipiell mittels zwei- oder mehrwertigen Kationen "gehärtete" Feststoffe durch Vermischen mit einem Fluid, welches derartige Komplexbildner enthält auf die im Endprodukt gewünschte Härte einstellen. Jedoch erfolgt die Erweichung durch diese Komplexbildner sehr rasch, so dass bereits bei einem unvollständigem Mischvorgang die Feststoffe schon vollständig erweicht sein können und bei Vollendung des Mischvorganges eine signifikante Anzahl an Feststoff bereits verformt, zerstört oder aufgeplatzt sind und daher kein optisch ansprechendes Produkt erhalten werden kann. Zudem erfolgt eine frühzeitige Vermischung, welche ungewünscht ist. Folglich sind bisherige Mischverfahren ungenügend.

Der Stand der Technik stellt sich daher als Problem dar.

KURZBESCHREIBUNG DER ERFINDUNG

Überraschend wurde nun gefunden, dass dieses Problem durch den Gegenstand der anliegenden Schutzansprüche gelöst werden konnte.

In einer Ausführungsform stellt die Neuerung ein Mischverfahren zur Verfügung, das Mischverfahren aufweisend die folgenden Schritte:

a) Bereitstellen eines kosmetischen Feststoffs (F),
b) Bereitstellen eines Fluids (S),

c) Zuführen des kosmetischen Feststoffs (F) und des Fluids (S) in einen statischen Mischer (M), und
d) Erhalten einer Mischung enthaltend den kosmetischen Feststoff (F) und das Fluid (S) aus dem statischen Mischer (M).

## KURZBESCHREIBUNG DER FIGUREN

Die Neuerung wird nun mit Bezug auf beispielhafte Ausführungsformen davon und die anliegenden Figuren in Einzelheiten beschrieben.
Es zeigt:

Fig. 1 ist eine Illustration zur Bestimmung der Kanalbreite.
Fig. 2A eine bevorzugte Vorrichtung mit der ein seitlicher Zulauf des Fluids (S) und des kosmetischen Feststoffes (F) realisiert werden kann.
Fig. 2B eine bevorzugte Vorrichtung mit der ein koaxialer Zulauf des Fluids (S) und des kosmetischen Feststoffes (F) realisiert werden kann.

## AUSFÜHRLICHE BESCHREIBUNG DER ERFINDUNG

In der vorliegenden Erfindung bezeichnen die Begriffe "Fluid" und "fluide Phase" Flüssigkeiten, Emulsionen und Suspensionen.

### Kosmetischer Feststoff (F)

Der kosmetische Feststoff (F) ist vorzugsweise partikelförmig.
Der kosmetische Feststoff (F) kann beispielsweise in rieselfähiger Form, als Agglomerat, Aufschlämmung, Pulver oder Gel in dem Verfahren eingesetzt werden. Vorzugsweise wird der kosmetische Feststoff (F) in Schritt a) in Form eines Fluids enthaltend den kosmetischen Feststoff (F) bereitgestellt, beispielsweise als Dispersion in einem Fluid bereitgestellt, üblicherweise als Suspension. Dieses Fluid unterscheidet sich üblicherweise von dem Fluid (S), welches in Schritt b) bereitgestellt wird.
Vorzugsweise enthält dieses Fluid ein Gel, insbesondere bevorzugt ein Hydrogel. Dieses Gel bzw. Hydrogel ist vorzugsweise ein nicht-newtonisches Gel. Insbesondere bevorzugt besteht das Fluid zu mindestens 75 Gew.%, mehr bevorzugt zu mindestens 90 Gew.% und am stärksten bevorzugt zu mindestens 95 Gew.% aus dem Gel bzw. dem Hydrogel. Derartige Gelstrukturen lassen sich bevorzugt durch den Einsatz von Polymeren ausbilden. Bevorzugt umfasst das Fluid ein oder mehrere Polymere ausgewählt aus der Gruppe bestehend aus Locust Bean Gum, Xanthan Gum, Gellan Gum, Carrageenan und Chondrus Chrispus Extract.
Gellan gum (Gellangummi) ist ein wasserlösliches anionisches Polysaccharid, das von dem Bakterium *Sphingomonas elodea* (ehemals *Pseudomonas elodea*) produziert wird. Im Grunde genommen ist Gellan gum ein lineares Polysaccharid, wobei die wiederkehrende Einheit des Polymers ein Tetrasaccharid ist, das aus einer L-Rhamnose, einer D-Glucuronsäure und zwei D-Glucoseeinheiten besteht, die mit Essigsäure und Glycerinsäure verestert sein können.
Es sind zwei verschiedene chemische Strukturen für Gellan Gum bekannt, die sich durch den Acylierungsgrad unterscheiden. Gellan Gum mit einem hohen Acylierungsgrad entsprechen der Formel (I), während Gellan Gums mit einem niedrigen Acylierungsgrad der Formel (II) entspricht.

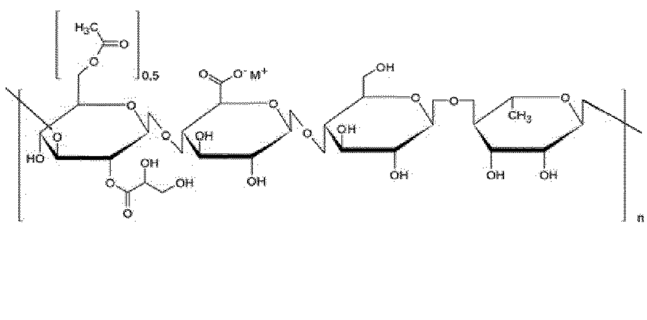

(I)

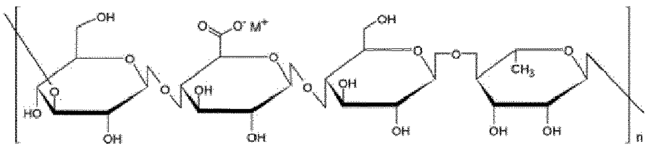

(II)

In den Formeln (I) und (II) steht n für eine ganzzahlige Zahl im Bereich von 2 bis 2000, vorzugsweise von 200 bis 450.

Für den Fall, dass das Fluid Gellan Gum umfasst, ist es weiterhin vorzuziehen, wenn das Gellan Gum eine Struktur gemäß Formel (II) aufweist. Weiterhin wird es erfindungsgemäß bevorzugt, wenn das Gellan Gum nach Formel (II) ein Molekulargewicht im Bereich von 200000 bis 300000 aufweist. Ein derartiges Gellan Gum kann unter dem Handelsnamen KELCOGEL® CG-LA von der Firma CP Kelco kommerziell erworben werden. Ist Gellan Gum enthalten, so beträgt der Anteil von Gellan Gum vorteilhaft von 0,01 bis 1,5 Gew.-%, weiterhin bevorzugt von 0,02 bis 0,5 Gew.-% und insbesondere bevorzugt von 0,05 bis 0,2 Gew.-% beträgt, bezogen auf das Gesamtgewicht des Fluids ohne den kosmetischen Feststoff.

Es ist erfindungsgemäß von Vorteil, wenn der Gesamtanteil des mindestens einem Polymers gewählt aus der Gruppe Locust Bean Gum, Xanthan Gum, Gellan Gum, Carrageenan und Chondrus Chrispus Extract von 0,1 bis 3 Gew.-%, weiterhin bevorzugt von 0,25 bis 2 Gew.-% und insbesondere bevorzugt von 0,4 bis 1,5 Gew.-% beträgt, bezogen auf das Gesamtgewicht des Fluids ohne den kosmetischen Feststoff.

Es ist besonders bevorzugt, wenn das Fluid mindestens Xanthan Gum und/oder Locust Bean Gum enthalten sind. Sind sowohl Xanthan Gum als auch Locust Bean Gum enthalten, so beträgt das Gewichtsverhältnis von Xanthan Gum zu Locust Bean Gum im Bereich von 2,0:1,0 bis 1,0:2,0, bevorzugt von 1,5:1,0 bis 1,0:1,5 und insbesondere bevorzugt von 1,2:1,0 bis 1,0:1,2 liegt.

Weiterhin ist es ebenfalls vorteilhaft im Sinne der vorliegenden Erfindung, wenn zusätzlich Natriumhyaluronat im Fluid enthalten ist, wobei der Anteil von Natriumhyaluronat bevorzugt von 0,01 bis 1 Gew.-%, weiterhin bevorzugt 0,05 bis 0,7 Gew.-% und insbesondere bevorzugt von 0,2 bis 0,5 Gew.-% beträgt, bezogen auf das Gesamtgewicht des Fluids ohne den kosmetischen Feststoff. Sofern Natriumhyaluronat enthalten ist, ist es insbesondere bevorzugt, wenn das Natriumhyaluronat ein Molekulargewicht im Bereich von 30000 Da bis 60000 Da aufweist.

Vorteilhaft umfasst das Fluid Wasser. Der Anteil von Wasser beträgt vorteilhaft von 70 bis 90 Gew.-%, bevorzugt von 75 Gew.-% bis 88 Gew.-% und insbesondere bevorzugt von 80 bis 86 Gew.-%, bezogen auf das Gesamtgewicht des Fluids ohne den kosmetischen Feststoff.

Ferner ist es vorteilhaft im Sinne der vorliegenden Erfindung, wenn das Fluid Glycerin enthält, wobei der Anteil von Glycerin von 5,0 bis 15 Gew.-%, bevorzugt von 7,5 bis 14 Gew.-% und insbesondere bevorzugt 8,0 bis 11 Gew.-%, bezogen auf das Gesamtgewicht des Fluids ohne den kosmetischen Feststoff, beträgt.

Weiterhin ist es vorteilhaft im Sinne der vorliegenden Erfindung, wenn das Fluid mindestens einen Lösungsvermittler umfasst, welcher weiterhin bevorzugt PEG-40 Hydrogenated Castor Oil (hydriertes Rizinusöl) ist. Ist PEG-40 Hydrogenated Castor Oil enthalten, ist es weiterhin bevorzugt, wenn der Anteil von PEG-40 Hydrogenated Castor Oil von 0,1 bis 0,8 Gew.-%, bezogen auf das Gesamtgewicht des Fluids ohne den kosmetischen Feststoff, beträgt.

In einer Ausführungsform der Erfindung umfasst das Fluid Polyvinylalcohol, Poly(meth)acrylat, Polyvinylpyrrolidon, Mischungen hiervon und/oder Copolymere hiervon.

In einer anderen, bevorzugten Ausführungsform umfasst das Fluid kein Polyvinylalcohol, Polyvinylpyrrolidon sowie keine Homo- oder Copolymere von Acrylsäure.

In einer besonders bevorzugten Ausführungsform umfasst das Fluid, bezogen auf das Gesamtgewicht des Fluids ohne den kosmetischen Feststoff:

- von 70 bis 90 Gew.-%, bevorzugt von 75 Gew.-% bis 88 Gew.-% und insbesondere bevorzugt von 80 bis 86 Gew.-% Wasser;
- von 5,0 bis 15 Gew.-%, bevorzugt von 7,5 bis 14 Gew.-% und insbesondere bevorzugt 8,0 bis 11 Gew.-% Glycerin;
- optional Xanthan Gum und Locust Bean Gum, wobei das Gewichtsverhältnis von Xanthan Gum zu Locust Bean Gum im Bereich von 2,0:1,0 bis 1,0:2,0, bevorzugt von 1,5:1,0 bis 1,0:1,5 und insbesondere bevorzugt von 1,2:1,0 bis 1,0:1,2;
- von 0,01 bis 1,5 Gew.-%, weiterhin bevorzugt von 0,02 bis 0,5 Gew.-% und insbesondere bevorzugt von 0,05 bis 0,2 Gew.-% Gellan Gum;
- optional von 0,1 bis 0,8 Gew.-% PEG-40 Hydrogenated Castor Oil (hydriertes Rizinusöl);
- optional von 0,01 bis 1 Gew.-%, weiterhin bevorzugt 0,05 bis 0,7 Gew.-% und insbesondere bevorzugt von 0,2 bis 0,5 Gew.-% Natriumhyaluronat,

wobei der Gesamtanteil von Locust Bean Gum, Xanthan Gum, Gellan Gum, Carrageenan und Chondrus Chrispus Extract von 0,1 bis 3 Gew.-%, weiterhin bevorzugt von 0,25 bis 2 Gew.-% und insbesondere bevorzugt von 0,4 bis 1,5 Gew.-% beträgt und wobei, vorzugsweise das Fluid kein Polyvinylalcohol, Polyvinylpyrrolidon sowie keine Homo- oder Copolymere von Acrylsäure umfasst.

Bevorzugte Varianten, Ausführungsformen und Merkmale der Bestandteile die in dieser Erfindung beschrieben sind, sind auch bevorzugte Varianten, Ausführungsformen und Merkmale dieser besonders bevorzugten Ausführungsform.

Vorteilhaft umfasst das Fluid kein Natriumcitrat und kein EDTA, vorzugsweise keine Citrate einwertiger Kationen und kein EDTA, insbesondere bevorzugt keine nichtpolymeren Komplexbildner für Calciumionen und am meisten bevorzugt keine nichtpolymeren Komplexbildner für zwei- oder mehrwertige Metallkationen. In der vorliegenden Erfindung werden als nichtpolymere Komplexbildner, Komplexbildner mit einem Molekulargewicht von nicht mehr als 1000 g/mol, vorzugsweise von nicht mehr als 500 g/mol angesehen.

Vorteilhaft weist das Fluid eine dynamische Viskosität $\eta$, bestimmt bei einer Temperatur von 25°C, von 1000 bis 6000 mPa·s, vorzugsweise 1500 bis 4500 mPa·s und am stärksten bevorzugt 1800 bis 2500 mPa·s aufweist. Die Messung der Viskosität ist im experimentellen Teil beschrieben.

Vorzugsweise beträgt der Anteil des kosmetischen Feststoffes, bezogen auf das Gesamtgewicht des Fluids, inklusive des kosmetischen Feststoffes (F) in Schritt a), 10,0 bis 50,0 Gew.%, mehr bevorzugt 20,0 bis 40,0 Gew.%.

Vorzugsweise beträgt das Gewichtsverhältnis zwischen dem Fluid und dem kosmetischen Feststoff 1:9 bis 1:1, vorzugsweise 1:5 bis 2:3.

Falls der kosmetische Feststoff (F) in Form eines Fluids enthaltend den kosmetischen Feststoff (F) bereitgestellt wird, beispielsweise als Dispersion in einem Fluid und dieses Fluid ein Gel enthält, beträgt die Summe der Gewichtsanteile des kosmetischen Feststoffes (F) und des Gels, bezogen auf das Gesamtgewicht des Fluids und des kosmetisches Feststoffes (F), beispielsweise der Dispersion, in Schritt a) vorzugsweise mindestens 50 Gew.%, insbesondere bevorzugt mindestens 65 Gew.% und am meisten bevorzugt mindestens 80 Gew.%.

Falls der kosmetische Feststoff (F) partikelförmig ist, enthält dieser vorzugsweise Mikrokapseln, stärker bevorzugt besteht der kosmetische Feststoff (F) zu mindestens 75 Gew.%, noch stärker bevorzugt zu mindestens 90 Gew.% und am stärksten bevorzugt zu 100% aus Mikrokapseln. Derartige Mikrokapseln weisen vorzugsweise mindestens zwei Phasen auf, stärker bevorzugt mindestens eine feste und mindestens eine fluide Phase, im Folgenden auch "flüssige Zubereitung" genannt. Strukturen derartiger Mikrokapseln sind aus dem Stand der Technik bekannt, beispielsweise können die Mikrokapseln Kern-Schale Mikrokapseln sein, deren Kern eine flüssige Zubereitung enthält, vorzugsweise aus diesem besteht. Diese flüssige Zubereitung kann eine oder mehrere Phasen aufweisen, beispielsweise eine Dispersion, wie eine Emulsion oder Suspension. Bevorzugt weist die flüssige Zubereitung enthaltend in dem Kern der Kern-Schale Mikrokapseln nur eine Phase auf, vorzugsweise eine Ölphase, wobei diese Ölphase vorzugsweise ein kosmetisches Öl enthält, oder daraus besteht. Diese flüssige Zubereitung ist nicht besonders beschränkt und kann prinzipiell jedes Fluid bzw. kosmetisches Fluid enthalten, vorzugsweise daraus bestehen. Üblicherweise wird ein Fluid bzw. kosmetisches Fluid verwendet, welches beispielsweise gegen erhöhte Temperatur, Licht, Feuchtigkeit, Sauerstoff, Reaktion mit anderen Inhaltsstoffen einer kosmetischen Zusammensetzung oder einer Kombination hiervon geschützt werden sollen. Zudem kann eine fluide Phase als Feststoff verarbeitet werden, wodurch die Handhabung erleichtert wird. Ferner kann eine Entmischung zweier oder mehrere fluider Phasen verhindert werden.

Die feste Phase der Mikrokapseln, im Falle von Kern-Schale Mikrokapseln üblicherweise die Schale der Mikrokapseln, enthält ein, vorzugsweise besteht zu mindestens 90 Gew.%, mehr bevorzugt zu 100 Gew.% aus einem, Material, welches für die Ummantelung von Fluiden und/oder festen Stoffen geeignet ist. Beispiele für derartige Materialien sind Proteine, wie z.B. Gelatine; Celluloseether; synthetische und natürliche Polymere, insbesondere Polysaccharide, wie z.B. Alginate und Pektine.

Eine besonders vorteilhafte Ausführungsform der Erfindung ist dadurch gekennzeichnet, dass die erfindungsgemäßen Mikrokapseln eine Hülle enthaltend ein Pektin und/oder Alginat, vorzugsweise Alginat, eines oder mehrerer zwei- oder mehrwertiger, üblicherweise zweiwertiger, Kationen, beispielsweise Erdalkaliionen, vorzugweise Calcium, beispielsweise Calciumalginat und einen flüssigen Kern, der vollständig von der Hülle umschlossen ist, aufweisen, wobei der Kern vorzugsweise eine Ölphase aufweist. Entsprechend enthalten bevorzugte Mikrokapseln mindestens ein flüssiges Öl. Die Mikrokapseln der Erfindung enthalten vorteilhaft ausschließlich einen einzigen flüssigen Kern.

Vorteilhaft enthält die Ölphase der erfindungsgemäßen Mikokapseln ein oder mehrere Öleausgewählt aus der Gruppe Octyldodecanol, Dicaprylylether, Caprylic/Capric-Triglyceride, Dimeticone, C12-15 Alkylbenzoat, Dicaprylylcarbonat; 2-Ethylhexylcocoat, Octylcocoate, Cetearyl-Isononanoate, Isopropylpalmitate und Squalan, insbesondere bevorzugt enthält die Ölphase der erfindungsgemäßen Mikrokapseln Octyldodecanol. Weiterhin ist es vorteilhaft, wenn die Ölphase der Mikrokapseln mindestens ein pflanzliches Öl, wie bevorzugt Arganöl, Aprikosenöl, Sonnenblumenöl, Nachtkerzenöl, Olivenöl, Mandelöl und/oder Jojobaöl, enthält. Weiterhin ist es vorteilhaft, wenn die Ölphase der Mikrokapseln Farbstoffe und/oder Mica enthält. Auf diese Weise können optisch ansprechende Mikrokapseln erhalten werden.

Die Herstellung von Mikrokapseln ist aus dem Stand der Technik bekannt, üblicherweise werden die Mikrokapseln mittels eines Extrusionsverfahrens hergestellt. Beispiele für derartige Verfahren sind Vibrationsverfahren, coaxial air-flow-Verfahren, elektrostatische Verfahren und Verfahren unter Verwendung eines jet cutters. Normalerweise haben die Herstellungsverfahren von Mikrokapseln gemein, dass die Mikrokapseln in einem vertikalen Verfahren hergestellt werden und dabei in ein Fluid eingetragen werden. Dieses Fluid ist üblicherweise ein Aushärtebad. In diesem Aushärtebad werden die Mikrokapseln beispielsweise durch physikalische Prozesse wie Abkühlung/Erhitzung und/oder durch chemische Reaktionen, wie z.B. mittels Gelierung, beispielsweise durch die oben genannten zwei- und/oder mehrwertigen Kationen, verfestigt. Falls beispielsweise Polysaccharide, wie Pektin und/oder Alginat, insbesondere Alginat, verwendet wird/werden, enthält das Aushärtebad eine, vorzugsweise besteht aus einer, Lösung von zwei- und/oder mehrwertigen Kationen, vorzugsweise Calciumionen.

Die erfindungsgemäßen Feststoffe/Mikrokapseln werden erfindungsgemäß vorteilhaft mittels Coextrusion aus einer dualen Düse erhalten werden. In diesem Verfahren weist die Zuleitung zur Düse ein inneres Rohr auf, welches sich innerhalb eines äußeren Rohres befindet. Entsprechend wird durch das innere Rohr die spätere Ölphase der Mikrokkapseln gepumpt und durch das äußere Rohr die eine wässrige Zusammensetzung enthaltend Natriumalginat. An der Düse enden beide Zuleitungsrohre, so dass bei der Ausgabe die Zusammensetzung des innen Rohrs von der Zusammensetzung des äußeren Rohres umgeben wird.

Es sind verschiedene Verfahren bekannt, die eine tropfenförmige Ausgabe ermöglichen. So kann die Tropfenbildung Vibrationen oder das Durchleiten eines Gas- oder Dampfstroms in der Nähe der Düse beinhalten. Bei Bildung der Tropfen sollte sichergestellt sein, dass die Ölphase im Tropfen vollständig von der Zusammensetzung enthaltend Natriumalginat umgegeben ist. Ein mögliches Verfahren ist beispielsweise in US 9,259,030 B2 beschrieben. Dort wird eine permanente Kraft über definierte Frequenzen und Amplituden an die Düsen angelegt, was dazu führt, dass sich aus dem Flüssigkeitsstrahl Tropfen wie vorstehend beschrieben bilden.

Bei Eintritt der Tropfen in ein wässriges Härtungsmedium, welches polyvalente Metallionen, insbesondere Calciumionen, enthält, geliert die äußere Phase des Tropfens, so dass sich kugelförmige Mikrokapseln mit einer inneren Ölphase ausbilden.

Die Schale der bevorzugten Kern-Schale Mikrokapseln weist vorzugsweise eine maximale Schichtdicke von weniger als 0,6 mm, mehr bevorzugt von weniger als 0,4 mm und insbesondere von weniger als 0,2 mm auf.

Der kosmetische Feststoff (F), wie beispielsweise die bevorzugten Kern-Schale Mikrokapseln, weist/weisen in Schritts a) vorzugsweise eine mittlere Bruchkraft von 0,20 bis 0,60 N auf, insbesondere bevorzugt von 0,30 N bis 0,50 N auf.

Der kosmetische Feststoff (F) bzw. die Mikrokapseln weisen vorzugsweise einen Feret-Durchmesser von 0,5 bis 20 mm, insbesondere bevorzugt von 1,0 bis 5,0 mm und am meisten bevorzugt von 1,4 bis 3,4 mm auf.

Der kosmetische Feststoff (F) bzw. die Mikrokapseln sind üblicherweise schersensitiv.

Falls der kosmetische Feststoff (F) als Dispersion in einem Fluid bereitgestellt wird, enthält dieses Fluid vorzugsweise Fluid des Aushärtebades, insbesondere bevorzugt besteht dieses Fluid aus Fluid des Aushärtebades.

Falls der kosmetische Feststoff (F) in Form eines Fluids enthaltend den kosmetischen Feststoff (F) bereitgestellt wird, beispielsweise als Dispersion in einem Fluid bereitgestellt wird, ist dieses Fluid üblicherweise frei von Stoffen, welche ein Aushärten der Mikrokapseln verlangsamt und/oder verhindert. Vorzugsweise enthält dieses Fluid kein Natriumcitrat und kein EDTA, vorzugsweise keine Citrate einwertiger Kationen und kein EDTA, insbesondere bevorzugt keine nichtpolymeren Komplexbildner für Calciumionen und am meisten bevorzugt keine nichtpolymeren Komplexbildner für zwei- oder mehrwertige Metallkationen..

Falls der kosmetische Feststoff (F) in Form eines Fluids enthaltend den kosmetischen Feststoff (F) bereitgestellt wird, beispielsweise als Dispersion in einem Fluid bereitgestellt wird, beträgt die dynamische Viskosität $\eta$ des Fluids enthaltend den kosmetischen Feststoff (F), bestimmt bei einer Temperatur von 25°C, vorzugsweise 1000 bis 6000 mPa·s, stärker bevorzugt 1500 bis 4500 mPa·s und am stärksten bevorzugt 1800 bis 2500 mPa·s.

Falls der kosmetische Feststoff (F) in Form eines Fluids enthaltend den kosmetischen Feststoff (F) bereitgestellt wird, beispielsweise als Dispersion in einem Fluid bereitgestellt wird, beträgt die dynamische Viskosität $\eta$ des Fluids, beispielsweise des Dispersionsmediums vorzugsweise eine dynamische Viskosität $\eta$, bestimmt bei einer Temperatur von 25°C, von 1000 bis 6000 mPa·s, stärker bevorzugt 1500 bis 4500 mPa·s und am stärksten bevorzugt 1800 bis 2500 mPa·s.

Erfindungsgemäß ist es vorteilhaft, die hergestellten Mikrokapseln in einer Transportlösung zu lagern. Dazu werden die Mikrokapseln nach Herstellung aus dem Härterbad entnommen und einer Transportlösung zugefügt. Die Transportlösung umfasst vorteilhaft mindestens 90 Gew.-% Wasser, bezogen auf das Gesamtgewicht der Transportlösung. Ferner ist es vorteilhaft, wenn die Transportlösung keine Calciumionen, insbesondere keine zwei- oder mehrwertigen Metallionen umfasst. Ferner ist es vorteilhaft, wenn die Transportlösung kein Natriumcitrat und kein EDTA, vorzugsweise keine Citrate einwertiger Kationen und kein EDTA, insbesondere bevorzugt keine nichtpolymeren Komplexbildner für Calciumionen und am meisten bevorzugt keine nichtpolymeren Komplexbildner für zwei- oder mehrwertige Metallkationen.. Vorteilhaft umfasst die Transportlösung ein oder mehrere Inhaltstoffe gewählt aus der Gruppe Methylpropanediol, 1,2-Hexandiol und/oder Phenoxyethanol. Vorteilhaft beträgt das Gewichtsverhältnis von Mikrokapseln zur

Transportlösung von 50:50 bis 90:10, bevorzugt von 60:40 bis 80:20 und insbesondere bevorzugt von 65:35 bis 75:25.

Das Fluid enthaltend die erfindungsgemäßen Mikrokapseln kann hergestellt werden, indem die Transportlösung enthaltend die Mikrokapseln im Fluid suspendiert werden. Vorteilhaft beträgt der Anteil der Mikrokapseln in der Mischung aus Transportlösung, Fluid und Mikrokapseln 20 Gew.-% bis 50 Gew.-% bezogen auf das Gesamtgewicht aus Transportlösung, Fluid und Mikrokapseln. Entsprechend wird erfindungsgemäß vorteilhaft ein Gel/Fluid enthaltend die erfindungsgemäßen Mikrokapseln erhalten.

Vorteilhaft weist das Fluid eine dynamische Viskosität $\eta$, bestimmt bei einer Temperatur von 25°C, von 1000 bis 6000 mPa·s, stärker bevorzugt 1500 bis 4500 mPa·s und am stärksten bevorzugt 1800 bis 2500 mPa·s auf.

Die Förderung des kosmetischen Feststoffes (F), insbesondere, wenn dieser in Form eines Fluids enthaltend den kosmetischen Feststoff (F) bereitgestellt wird, beispielsweise als Dispersion in einem Fluid bereitgestellt wird, erfolgt vorzugsweise über pulsationsarme Pumpen, wie beispielsweise Exzenterschneckenpumpen, Schraubenspindelpumpen, Sinusoidalpumpen oder druckbeaufschlagte Behälter.

Fluid (S)

Das Fluid (S) enthält vorzugsweise ein Gel, insbesondere bevorzugt ein Hydrogel. Dieses Gel bzw. Hydrogel ist vorzugsweise ein nicht-newtonisches Gel. Insbesondere bevorzugt besteht das Fluid zu mindestens 75 Gew.%, mehr bevorzugt zu mindestens 90 Gew.% und am stärksten bevorzugt zu mindestens 95 Gew.% aus dem Gel bzw. dem Hydrogel. Vorzugsweise enthält das Fluid (S) ein Gel, insbesondere bevorzugt ein Hydrogel. Derartige Gelstrukturen lassen sich bevorzugt durch den Einsatz von Polymeren ausbilden. Bevorzugt umfasst das Fluid (S) ein oder mehrere Polymere ausgewählt aus der Gruppe bestehend aus Locust Bean Gum, Xanthan Gum, Gellan Gum, Carrageenan und Chondrus Chrispus Extract.

Gellan gum (Gellangummi) ist ein wasserlösliches anionisches Polysaccharid, das von dem Bakterium *Sphingomonas elodea* (ehemals *Pseudomonas elodea*) produziert wird. Im Grunde genommen ist Gellan gum ein lineares Polysaccharid, wobei die wiederkehrende Einheit des Polymers ein Tetrasaccharid ist, das aus einer L-Rhamnose, einer D-Glucuronsäure und zwei D-Glucoseeinheiten besteht, die mit Essigsäure und Glycerinsäure verestert sein können.

Es sind zwei verschiedene chemische Strukturen für Gellan Gum bekannt, die sich durch den Acylierungsgrad unterscheiden. Gellan Gum mit einem hohen Acylierungsgrad entsprechen der Formel (III), während Gellan Gums mit einem niedrigen Acylierungsgrad der Formel (IV) entspricht.

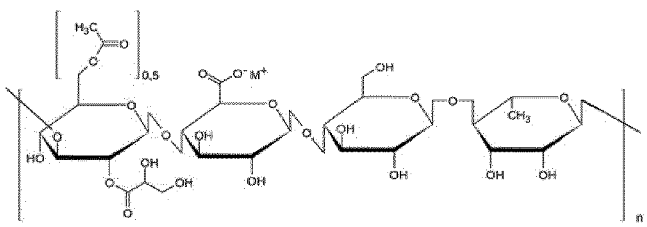

(III)

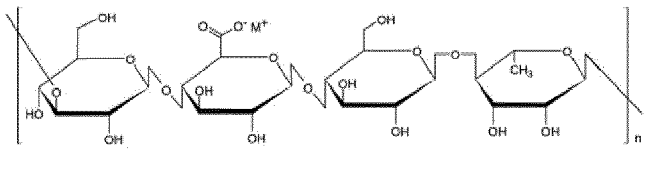

(IV)

In den Formeln (III) und (IV) steht n für eine ganzzahlige Zahl im Bereich von 2 bis 2000, vorzugsweise von 200 bis 450.

Für den Fall, dass das Fluid (S) Gellan Gum umfasst, ist es weiterhin vorzuziehen, wenn das Gellan Gum eine Struktur gemäß Formel (IV) aufweist. Weiterhin wird es erfindungsgemäß bevorzugt, wenn das Gellan Gum nach Formel (IV) ein Molekulargewicht im Bereich von 200000 bis 300000 aufweist. Ein derartiges Gellan Gum kann unter dem Handelsnamen KELCOGEL® CG-LA von der Firma CP Kelco kommerziell erworben werden. Ist Gellan Gum enthalten, so beträgt der Anteil von Gellan Gum vorteilhaft von 0,01 bis 1,5 Gew.-%, weiterhin bevorzugt von 0,02 bis 0,5 Gew.-% und insbesondere bevorzugt von 0,05 bis 0,2 Gew.-% beträgt, bezogen auf das Gesamtgewicht des Fluids (S).

Es ist erfindungsgemäß von Vorteil, wenn der Gesamtanteil des mindestens einem Polymers gewählt aus der Gruppe Locust Bean Gum, Xanthan Gum, Gellan Gum, Carrageenan und Chondrus Chrispus Extract von 0,1 bis 3

Gew.-%, weiterhin bevorzugt von 0,25 bis 2 Gew.-% und insbesondere bevorzugt von 0,4 bis 1,5 Gew.-% beträgt, bezogen auf das Gesamtgewicht des Fluids (S).

Es ist besonders bevorzugt, wenn das Fluid (S) mindestens Xanthan Gum und/oder Locust Bean Gum enthalten sind. Sind sowohl Xanthan Gum als auch Locust Bean Gum enthalten, so beträgt das Gewichtsverhältnis von Xanthan Gum zu Locust Bean Gum im Bereich von 2,0:1,0 bis 1,0:2,0, bevorzugt von 1,5:1,0 bis 1,0:1,5 und insbesondere bevorzugt von 1,2:1,0 bis 1,0:1,2 liegt.

Weiterhin ist es ebenfalls vorteilhaft im Sinne der vorliegenden Erfindung, wenn zusätzlich Natriumhyaluronat im Fluid (S) enthalten ist, wobei der Anteil von Natriumhyaluronat bevorzugt von 0,01 bis 1 Gew.-%, weiterhin bevorzugt 0,05 bis 0,7 Gew.-% und insbesondere bevorzugt von 0,2 bis 0,5 Gew.-% beträgt, bezogen auf das Gesamtgewicht des Fluids (S). Sofern Natriumhyaluronat enthalten ist, ist es insbesondere bevorzugt, wenn das Natriumhyaluronat ein Molekulargewicht im Bereich von 30000 Da bis 60000 Da aufweist.

Vorteilhaft umfasst das Fluid (S) Wasser. Der Anteil von Wasser beträgt vorteilhaft von 70 bis 90 Gew.-%, bevorzugt von 75 Gew.-% bis 88 Gew.-% und insbesondere bevorzugt von 80 bis 86 Gew.-%, bezogen auf das Gesamtgewicht des Fluids (S).

Ferner ist es vorteilhaft im Sinne der vorliegenden Erfindung, wenn das Fluid (S) Glycerin enthält, wobei der Anteil von Glycerin von 5,0 bis 15 Gew.-%, bevorzugt von 7,5 bis 14 Gew.-% und insbesondere bevorzugt 8,0 bis 11 Gew.-%, bezogen auf das Gesamtgewicht des Fluids (S).

Weiterhin ist es vorteilhaft im Sinne der vorliegenden Erfindung, wenn das Fluid (S) mindestens einen Lösungsvermittler umfasst, welcher weiterhin bevorzugt PEG-40 Hydrogenated Castor Oil (hydriertes Rizinusöl) ist. Ist PEG-40 Hydrogenated Castor Oil enthalten, ist es weiterhin bevorzugt, wenn der Anteil von PEG-40 Hydrogenated Castor Oil von 0,1 bis 0,8 Gew.-%, bezogen auf das Gesamtgewicht des Fluids (S).

In einer Ausführungsform der Erfindung umfasst das Fluid (S) Polyvinylalcohol, Poly(meth)acrylat, Polyvinylpyrrolidon, Mischungen hiervon und/oder Copolymere hiervon.

In einer anderen, bevorzugten Ausführungsform umfasst das Fluid (S) kein Polyvinylalcohol, Polyvinylpyrrolidon sowie keine Homo- oder Copolymere von Acrylsäure.

Das Fluid (S) kann weitere kosmetische Inhaltsstoffe enthalten, diese können beispielsweise fest und/oder flüssig sein.

In einer bevorzugten Ausführungsform enthält das Fluid (S) einen oder mehrere nichtpolymere Komplexbildner für zwei- oder mehrwertige Metallkationen, vorzugsweise einen oder mehrere nichtpolymere Komplexbildner für Calciumionen, stärker bevorzugt Citrate einwertiger Kationen und/oder EDTA, insbesondere bevorzugt Natriumcitrat. In einer bevorzugten Variante dieser Ausführungsform beträgt die Gesamtmenge an nichtpolymeren Komplexbildnern für zwei- oder mehrwertige Metallkationen, nichtpolymeren Komplexbildnern für Calciumionen, Citraten einwertiger Kationen und EDTA bzw. Natriumcitrat vorzugsweise 0,1 bis 1,0 Gew.% bezogen auf das Gesamtgewicht des Fluids (S)

In jeder Alternative dieser bevorzugten Variante dieser Ausführungsform beträgt die Gesamtmenge an nichtpolymeren Komplexbildnern mit einem Molekulargewicht bis 1000 g/mol vorzugsweise jeweils 0,1 bis 1,0 Gew.% bezogen auf das Gesamtgewicht des Fluids (S).

Diese bevorzugte Ausführungsform des Fluids (S) ist insbesonders bevorzugt, wenn die erfindungsgemäßen Mikrokapseln eine Hülle enthaltend ein Pektin und/oder Alginat, vorzugsweise Alginat, eines oder mehrerer zwei- oder mehrwertiger, üblicherweise zweiwertiger, Kationen, beispielsweise Erdalkaliionen, vorzugweise Calcium, beispielsweise Calciumalginat und einen flüssigen Kern, der vollständig von der Hülle umschlossen ist, aufweisen.

In einer besonders bevorzugten Ausführungsform umfasst das Fluid (S), bezogen auf das Gesamtgewicht des Fluids (S):

- von 70 bis 90 Gew.-%, bevorzugt von 75 Gew.-% bis 88 Gew.-% und insbesondere bevorzugt von 80 bis 86 Gew.-% Wasser;
- von 5,0 bis 15 Gew.-%, bevorzugt von 7,5 bis 14 Gew.-% und insbesondere bevorzugt 8,0 bis 11 Gew.-% Glycerin;
- optional Xanthan Gum und Locust Bean Gum, wobei das Gewichtsverhältnis von Xanthan Gum zu Locust Bean Gum im Bereich von 2,0:1,0 bis 1,0:2,0, bevorzugt von 1,5:1,0 bis 1,0:1,5 und insbesondere bevorzugt von 1,2:1,0 bis 1,0:1,2;
- von 0,01 bis 1,5 Gew.-%, weiterhin bevorzugt von 0,02 bis 0,5 Gew.-% und insbesondere bevorzugt von 0,05 bis 0,2 Gew.-% Gellan Gum;
- optional von 0,1 bis 0,8 Gew.-% PEG-40 Hydrogenated Castor Oil (hydriertes Rizinusöl);
- optional von 0,01 bis 1 Gew.-%, weiterhin bevorzugt 0,05 bis 0,7 Gew.-% und insbesondere bevorzugt von 0,2 bis 0,5 Gew.-% Natriumhyaluronat; und
- von 0,1 bis 1,0 Gew.% eines oder mehrerer nichtpolymerer Komplexbildner für zwei- oder mehrwertige Metallkationen, vorzugsweise eines oder mehrerer nichtpolymere Komplexbildner für Calciumionen, stärker bevorzugt Citrate einwertiger Kationen und/oder EDTA, insbesondere bevorzugt Natriumcitrat,

wobei der Gesamtanteil von Locust Bean Gum, Xanthan Gum, Gellan Gum, Carrageenan und Chondrus Chrispus Extract von 0,1 bis 3 Gew.-%, weiterhin bevorzugt von 0,25 bis 2 Gew.-% und insbesondere bevorzugt von 0,4 bis 1,5 Gew.-% beträgt und wobei, vorzugsweise das Fluid (S) kein Polyvinylalcohol, Polyvinylpyrrolidon sowie keine Homo- oder Copolymere von Acrylsäure umfasst.

Bevorzugte Varianten, Ausführungsformen und Merkmale der Bestandteile die in dieser Erfindung beschrieben sind, sind auch bevorzugte Varianten, Ausführungsformen und Merkmale dieser besonders bevorzugten Ausführungsform.

Das Fluid (S) weist vorzugsweise eine dynamische Viskosität $\eta$, bestimmt bei einer Temperatur von 25°C, von 1000 bis 6000 mPa·s, stärker bevorzugt 1500 bis 4500 mPa·s und am stärksten bevorzugt 1800 bis 2500 mPa·s auf.

Die Förderung des Fluids (S) erfolgt vorzugsweise über pulsationsarme Pumpen, wie beispielsweise Exzenterschneckenpumpen, Schraubenspindelpumpen, Sinusoidalpumpen oder druckbeaufschlagte Behälter.

Falls der kosmetische Feststoff (F) in Form eines Fluids enthaltend den kosmetischen Feststoff (F) bereitgestellt wird, beispielsweise als Dispersion in einem Fluid bereitgestellt wird, beträgt die Differenz zwischen der dynamischen Viskosität $\eta$ des Fluids enthaltend den kosmetischen Feststoff (F) inklusive des kosmetischen Feststoffes (F), bestimmt bei einer Temperatur von 25°C, und der dynamischen Viskosität $\eta$ des Fluids (S), bestimmt bei einer Temperatur von 25°C, vorzugsweise zwischen -1000 und 1000 mPa·s, stärker bevorzugt zwischen -500 und 500 mPa·s und am meisten bevorzugt zwischen -250 und 250 mPa·s.

Statischer Mischer (M)

Ein statischer Mischer (manchmal auch als Statikmischer bezeichnet) ist eine Vorrichtung geeignet zum Mischen von Feststoffen, beispielsweise rieselfähigen Feststoffen und/oder Fluiden, in der allein die Strömungsbewegung die Vermischung bewirkt und die Vermischung nicht mittels bewegter Elemente erfolgt. Ein statischer Mischer besteht üblicherweise aus strömungsbeeinflussenden Elementen in einem Rohr. Diese strömungsbeeinflussenden Elemente teilen den Stoffstrom auf und führen ihn in neuer Sequenzierung wieder zusammen, wodurch die Vermischung erreicht wird. Teile des statischen Mischers, die ausgebaut werden können, wenn der statische Mischer nicht in Betrieb ist, beispielsweise zu Reinigungszwecken, Wartungs- und/oder Kontrollzwecken sind keine bewegten Elemente über die eine Vermischung erfolgt.

Der statische Mischer (M) ist derart ausgelegt, dass der kosmetische Feststoff (F) den statischen Mischer (M) vollständig passieren kann, d.h. nicht an einer zu kleinen Öffnung o.ä. "steckenbleibt". Anders ausgedrückt, der statische Mischer weist Kanäle auf, deren Kanalbreite derart ausgelegt ist, dass der kosmetische Feststoff (F) durch diese gelangen kann, ohne sich zu verformen.

Definitionsgemäß gibt die Kanalbreite die geringste messbare Breite x in einem Mischungskanal des Mischers an, wobei die Breite x immer im 90° Winkel zur jeweiligen Strömungsrichtung des Fluids angegeben ist. Dies ist in Fig. 1 gezeigt, wobei die gestrichelten Pfeile die jeweiligen Strömungsrichtungen angeben. Die Kanäle können in Bezug auf die Strömungsrichtung des Fluids vor dem Mischer in einem Winkel ($\alpha$) in dem Bereich von 0-85° angeordnet sein können. Der Winkel $\alpha$ kann ggf. über die Länge des Mischers variieren, beispielsweise bei einer helikalen Struktur. Ein Kanal kann definitionsgemäß durch Bauteile des Mischers alleine oder durch die Bauteile des Mischers und der Röhre begrenzt sein.

Der statische Mischers (M) weist vorzugsweise Kanäle auf, die eine Kanalbreite aufweisen, welche mehr als dem 2,0-fachen, vorzugsweise mindestens dem 3,0-fachen, vorzugsweise mindestens dem 4,0-fachen des Feret-Durchmessers des kosmetischen Feststoffes (F) entspricht. Üblicherweise weist der statische Mischers (M) keine Kanäle auf, die eine Kanalbreite aufweisen, welche dem 10,0-fachen des Feret-Durchmessers des kosmetischen Feststoffes (F) oder mehr entspricht.

Das Verhältnis der Länge des statischen Mischers gemessen von dem Beginn bis zum Ende aller vorhandenen strömungsbeeinflussenden Elementen zu dem durchschnittlichen Innenquerschnitt des statischen Mischers entlang dieser Länge ohne die darin enthaltenen strömungsbeeinflussenden Elemente beträgt vorzugsweise 1,0 bis 50, mehr bevorzugt 5,0 bis 35 und am stärksten bevorzugt 7,5 bis 25.

Als Innenquerschnitt wird die Querschnittsfläche des statischen Mischers angesehen, inklusive der darin befindlichen strömungsbeeinflussenden Elemente, jedoch exklusive der Außenwand und, falls vorhanden, daran angebrachter weiterer Vorrichtungen.

Der durchschnittliche Innenquerschnitt des statischen Mischers (M) gemessen von dem Beginn bis zum Ende aller vorhandenen strömungsbeeinflussenden Elemente beträgt vorzugsweise zwischen 5,0 bis 25 $cm^2$, mehr bevorzugt zwischen 7,5 und 20 $cm^2$.

Die Länge des statischen Mischers (M) gemessen von dem Beginn bis zum Ende aller vorhandenen strömungsbeeinflussenden Elementen beträgt vorzugsweise 10 bis 120 cm, mehr bevorzugt 20 bis 105 cm und am stärksten bevorzugt 30 bis 90 cm. Falls der statische Mischer (M) nicht linear ist, so ist die Länge die kürzeste Entfernung die ein Fluid von dem Beginn bis zum Ende aller vorhandenen strömungsbeeinflussenden Elemente innerhalb des statischen

Mischers (M) zurücklegt.

Der Flächenanteil der strömungsbeeinflussenden Elemente bezogen auf den Innenquerschnitt des statischen Mischers (M) entlang der Gesamtlänge des statischen Mischers (M) beträgt vorzugsweise weniger als 50%, insbesondere bevorzugt weniger als 25%. Als Gesamtlänge des statischen Mischers (M) wird die Länge von dem Beginn bis zu dem Ende aller vorhandenen strömungsbeeinflussenden Elementen angesehen. Die strömungsbeeinflussenden Elemente verringern die durchströmbare Querschnittsfläche des statischen Mischers, was zu einer höheren Durchflussgeschwindigkeit durch diese Querschnittsfläche führt und/oder die Durchflussgeschwindigkeit durch den statischen Mischer (M) insgesamt senkt. Dies kann, bei bestimmten Mischgütern u.U. vorteilhaft sein, in der vorliegenden Erfindung ist es jedoch nicht bevorzugt.

Der Innenquerschnitt des statischen Mischers (M) kann entlang der Gesamtlänge des statischen Mischers (M) variieren. Vorzugsweise ist der Innenquerschnitt des statischen Mischers (M) entlang der Gesamtlänge des statischen Mischers (M) identisch.

Der Innenquerschnitt des statischen Mischers (M) entlang der Gesamtlänge des statischen Mischers (M) ist vorzugsweise rechteckig, quadratisch, kreis- oder ellipsenförmig, besonders bevorzugt kreis- oder ellipsenförmig und insbesondere bevorzugt kreisförmig.

Die strömungsbeeinflussenden Elemente des statischen Mischers (M) sind üblicherweise wendel-, steg-, lamellen-, gitterförmig oder als eine Mischung hiervon ausgelegt. Vorzugsweise sind die strömungsbeeinflussenden Elemente des statischen Mischers (M) wendel-, steg-, lamellen- oder gitterförmig, insbesondere bevorzugt wendel- oder stegförmig. Mit anderen Worten, der statische Mischer weist vorzugsweise keine Kombination von zwei oder mehr der wendel-, steg-, lamellen- und gitterförmigen strömungsbeeinflussenden Elemente auf.

Ein statischer Mischer der wendelförmige strömungsbeeinflussenden Elemente, im Folgenden auch als "Wendeln" bezeichnet, aufweist, weist üblicherweise zwei oder mehr Wendeln auf, die in Durchflussrichtung durch den statischen Mischer (M) hintereinander abwechselnd rechts- und linksgängig angeordnet sind. Jede der Wendeln kann den Innenquerschitt des statischen Mischer in zwei bis sechs Kanäle aufteilen, üblicherweise teilt jedoch jede der Wendeln den Innenquerschitt des statischen Mischer in nur zwei Kanäle auf. Jede einzelne dieser Wendeln weist vorzugsweise einen Verdrehungswinkel von 120 bis 240°, stärker bevorzugt 150 bis 210°, noch stärker bevorzugt 170 bis 190°, wie beispielsweise ungefähr 180° auf. Die Enden benachbarter Wendeln treffen vorzugsweise unter einem Winkel von 60/(0.5·k) bis 120/(0.5·k)°, vorzugsweise 80/(0.5·k) bis 100/(0.5·k)°, wie beispielsweise ungefähr 90/k(0.5·k)° aufeinander, wobei k die Zahl der Kanäle ist, in welche die Wendel den Innenquerschitt des statischen Mischer aufteilt. Sollten benachbarte Wendeln den Innenquerschitt des statischen Mischer in eine unterschiedliche Anzahl von Kanälen aufweisen, so wird für den Wert k, die höhere der beiden Anzahlen angesetzt. Üblicherweise enthält ein statischer Mischer, der Wendeln aufweist, nicht mehr als 20 Wendeln, vorzugsweise nicht mehr als 15 Wendeln.

Im Folgenden sind bevorzugte Merkmale eines statischen Mischers enthaltend Wendeln aufgeführt.

Die Wendeln weisen jeweils vorzugweise ein Verhältnis ihrer Länge zu dem durchschnittlichen Innenquerschnitt des statischen Mischers (M) entlang dieser Länge von 2,5 $cm^{-1}$ bis 10 $cm^{-1}$, mehr bevorzugt von 3,5 $cm^{-1}$ bis 7,5 $cm^{-1}$ und am stärksten bevorzugt von 4,5 $cm^{-1}$ bis 6,0 $cm^{-1}$ auf. Falls der statische Mischer (M) einen kreisförmigen Innenquerschnitt aufweist und der Innenquerschnitt des statischen Mischers (M) entlang der Gesamtlänge des statischen Mischers (M) identisch ist, beträgt das Verhältnis der Länge jeder Wendel zu dem Innendurchmesser des statischen Mischers (M) vorzugsweise 0,5 bis 3,0, mehr bevorzugt 1,0 bis 2,5 und insbesondere bevorzugt 1,5 bis 2,0.

Die Distanz zwischen zwei benachbarten Wendeln, falls vorhanden, beträgt jeweils vorzugsweise nicht mehr als 5,0 cm, insbesondere bevorzugt nicht mehr als 2,0 cm, am stärksten bevorzugt nicht mehr als 0,1 cm. In einer besonders bevorzugten Variante sind die Wendeln so angeordnet, dass sich zwei benachbarte Wendeln jeweils berühren.

Die Wendeln können abgesehen von ihrer Rechts- bzw. Linksgängigkeit identisch oder untereinander verschieden sein. Eine rechtsgängige Wendel wird mit einer linksgängigen Wendel als identisch angesehen, wenn sie sich wie Bild und Spiegelbild verhalten.

Ein Beispiel für einen statischer Mischer der Wendeln enthält ist ein Kenics® Wendelmischer.

Stege sind üblicherweise plattenförmig, d.h. zwei der drei räumlichen Dimensionen des Steges sind deutlich größer als die dritte räumliche Dimension. Die beiden Flächen die von den zwei größeren Dimensionen aufgespannt werden, werden im Folgenden als "Grundflächen" bezeichnet. Diese beiden Grundflächen sind üblicherweise parallel.

Ein statischer Mischer, der stegförmige strömungsbeeinflussende Elemente, im Folgenden als "Stege" bezeichnet, aufweist, weist üblicherweise ein oder mehrere Gerüste, jeweils aufweisend zwei oder mehr Stege, auf. In einem derartigen Gerüst sind üblicherweise Stege hintereinander und gegeneinander geneigt angeordnet. Das Gerüst und die darin enthaltenen Stege sind üblicherweise derart angeordnet, dass eine Grundfläche jedes Steges der Strömungsrichtung durch den statischen Mischer (M), wobei als Strömungsrichtung die Strömungsrichtung ohne die darin enthaltenen strömungsbeeinflussenden Elemente angesehen wird, zugewandt ist. Hierbei verhält sich diese Strömungsrichtung nicht wie ein Lot auf eine Grundfläche jedes einzelnen Steges.

Im Folgenden werden bevorzugte Merkmale eines statischen Mischers enthaltend Stege beschrieben.

Der Schnittwinkel der Strömungsrichtung durch den statischen Mischer (M), wobei als Strömungsrichtung die

Strömungsrichtung ohne die darin enthaltenen strömungsbeeinflussenden Elemente angesehen wird, und jedem einzelnen Steg beträgt vorzugsweise zwischen 10 und 80°, mehr bevorzugt zwischen 20 und 65° und am meisten bevorzugt zwischen 30 und 45°. Der Schnittwinkel zwischen einer Gerade (hier der Strömungsrichtung) und einer Ebene (hier dem einzelnen Steg) ist der kleinste Winkel den eine beliebige Gerade enthaltend in der Ebene mit der Geraden (hier der Strömungsrichtung) bildet.

Üblicherweise sind alle Stege innerhalb eines Gerüstes derart angeordnet, dass das Gerüst derart zur Blickrichtung des Betrachters ausgerichtet werden kann, dass die Grundflächen aller in dem Gerüst vorhandenen Stege jeweils eine Gerade ausweisen, die zur Blickrichtung des Betrachters einen Schnittwinkel von nicht mehr als 10°, vorzugsweise nicht mehr als 5° aufweist, insbesondere bevorzugt weisen die Grundflächen aller in dem Gerüst vorhandenen Stege jeweils eine Gerade auf, die zur Blickrichtung des Betrachters parallel ist.

Innerhalb eines Gerüstes sind üblicherweise zwei oder mehrere Gruppen von Stegen angeordnet, normalerweise nicht mehr als sechs, in Strömungsrichtung durch den statischen Mischer (M) betrachtet, wobei als Strömungsrichtung die Strömungsrichtung ohne die darin enthaltenen strömungsbeeinflussenden Elemente angesehen wird, nebeneinander angeordnet. Jede Gruppe besteht vorzugsweise aus 2 bis 8, mehr bevorzugt 2 bis 6, Stegen, die, in Strömungsrichtung durch den statischen Mischer (M) betrachtet, wobei als Strömungsrichtung die Strömungsrichtung ohne die darin enthaltenen strömungsbeeinflussenden Elemente angesehen wird, hintereinander angeordnet sind, wobei diese Stege vorzugsweise parallel zueinander angeordnet sind und/oder der minimale Abstand zweier benachbarter Stege innerhalb einer Gruppe 5,0 mm bis 30 mm, vorzugsweise 5,0 mm bis 20 mm beträgt. Die Stege zweier benachbarter Gruppen weisen jeweils untereinander vorzugsweise einen Schnittwinkel von 45° bis 90°, mehr bevorzugt von 55° bis 90°, auf. Da sich die Stege üblicherweise nicht im geometrischen Sinn schneiden, wird zur Schnittwinkelbestimmung von den Ebenen ausgegangen, in denen eine Grundfläche der jeweiligen Stege liegt. Sollte bei der Bestimmung dieses Schnittwinkels mittels den beiden Grundflächen eines Steges verschiedene Ergebnisse resultieren, so ist der kleinere Wert ausschlaggebend.

Der statische Mischer (M) weist üblicherweise zwei oder mehr, vorzugsweise nicht mehr als fünf, Gerüste auf, die sich voneinander unterscheiden können oder identisch sind. Vorzugsweise sind alle Gerüste, falls vorhanden, identisch. Benachbarte Gerüste sind, in Strömungsrichtung durch den statischen Mischer (M) gesehen, wobei als Strömungsrichtung die Strömungsrichtung ohne die darin enthaltenen strömungsbeeinflussenden Elemente angesehen wird, üblicherweise zueinander verdreht angeordnet. Vorzugsweise sind zwei benachbarte Gerüste innerhalb des statischen Mischer (M), in Strömungsrichtung durch den statischen Mischer (M) gesehen, wobei als Strömungsrichtung die Strömungsrichtung ohne die darin enthaltenen strömungsbeeinflussenden Elemente angesehen wird um mindestens 45° zueinander verdreht, vorzugsweise um mindestens 60° zueinander verdreht angeordnet.

Beispiele für einen derartigen Mischer sind die Mischer der SMX- und SMXL-Produktfamilien der Firma Sulzer und die LPD- und LLPD-Produktfamilien der Firma Ross.

Statische Mischer mit lamellenförmigen strömungsbeeinflussenden Elementen weisen üblicherweise aufeinandergeschichtete geriffelte, bzw. gewellte Lamellen auf, die offene, sich kreuzende Kanäle bilden. Beispiele für derartige statische Mischer ist die SMV-Produktfamilie der Firma Sulzer.

Ein gitterförmiges strömungsbeeinflussendes Element ist üblicherweise aus regelmäßigen dreidimensionalen Gittern aufgebaut. Die Breite der gitterformenden Elemente und die Größe der Zellen lassen sich variieren. Üblicherweise weisen die gitterförmigen strömungsbeeinflussenden Elemente quaderförmige, bevorzugt kubische Zellen auf, insbesondere bevorzugt sind im Wesentlichen alle Zellen quaderförmig, noch stärker bevorzugt kubisch. Der Begriff "im Wesentlichen" wird hier verwendet, da die Zellen an der Innenwand des statischen Mischers, beispielsweise falls der Innenquerschnitt des statischen Mischers (M) entlang der Gesamtlänge des statischen Mischers (M) kreis- oder ellipsenförmig ist, u.U. nicht quaderförmig bzw. kubisch sein können. Falls vorhanden, weist der statische Mischer (M) vorzugsweise nur ein gitterförmiges strömungsbeeinflussendes Element auf.

Vorzugsweise weist der statische Mischer (M) wendelförmige oder stegförmige strömungsbeeinflussende Elemente auf, insbesondere bevorzugt wendelförmige strömungsbeeinflussende Elemente.

Es ist grundsätzlich möglich, in dem Verfahren gemäß der vorliegenden Erfindung einen weiteren statischen Mischer zu verwenden, und falls vorhanden, üblicherweise einen statischen Mischer (M) gemäß der vorliegenden Erfindung. Es ist jedoch bevorzugt, in dem Verfahren gemäß der vorliegenden Erfindung nur einen statischen Mischer zu verwenden.

Das Mischverfahren gemäß der vorliegenden Anmeldung weist ferner vorzugsweise den folgenden Schritt auf: e) Abfüllen der Mischung erhalten aus Schritt d), vorzugsweise in ein oder mehrere kosmetische Packmittel, wie beispielsweise in einen oder mehrere Dispenser, Tiegel, Pumpspender, insbesondere Pumpspender, wie beispielweise Schleppkolbenspender;

wobei, vorzugsweise, die mittlere Verweilzeit des kosmetischen Feststoffs (F) und des Fluids (S) zwischen dem Beginn von Schritt c) und dem Ende von Schritt e) nicht mehr als 45 Minuten, stärker bevorzugt nicht mehr als 30 Minuten, noch stärker bevorzugt nicht mehr als 20 Minuten und am stärksten bevorzugt nicht mehr als 15 Minuten beträgt. Mit anderen Worten, vorzugsweise ist die Vorrichtung zum Abfüllen der aus dem statischen Mischer erhaltenen Mischung direkt im

Anschluss an den statischen Mischer angeordnet.

Die Ausgabevorrichtung des kosmetischen Packmittels, falls vorhanden, ist vorzugsweise derart konfiguriert, dass der kosmetische Feststoff (F) bei Ausgabe durch die eintretenden Kräfte zerdrückt wird und mit dem Fluid (S) vermischt wird.

Vorzugsweise erfolgt das Zuführen des kosmetischen Feststoffs (F) bei konstanter Strömungsgeschwindigkeit und/oder bei einer Strömungsgeschwindigkeit von 0,25 bis 5,0 $l \cdot min^{-1}$, stärker bevorzugt von 0,50 bis 3,0 $l \cdot min^{-1}$ und am stärksten bevorzugt von 1,0 bis 2,0 $l \cdot min^{-1}$. Insbesondere bevorzugt erfolgt das Zuführen des kosmetischen Feststoffs (F) bei konstanter Strömungsgeschwindigkeit und bei einer Strömungsgeschwindigkeit von 0,25 bis 5,0 $l \cdot min^{-1}$, stärker bevorzugt von 0,50 bis 3,0 $l \cdot min^{-1}$ und am stärksten bevorzugt von 1,0 bis 2,0 $l \cdot min^{-1}$.

Vorzugsweise erfolgt das Zuführen des Fluids (S) bei konstanter Strömungsgeschwindigkeit und/oder bei einer Strömungsgeschwindigkeit von 0,25 bis 5,0 $l \cdot min^{-1}$, stärker bevorzugt von 0,50 bis 3,0 $l \cdot min^{-1}$ und am stärksten bevorzugt von 1,0 bis 2,0 $l \cdot min^{-1}$. Insbesondere bevorzugt erfolgt das Zuführen des Fluids (S) bei konstanter Strömungsgeschwindigkeit und bei einer Strömungsgeschwindigkeit von 0,25 bis 5,0 $l \cdot min^{-1}$, stärker bevorzugt von 0,50 bis 3,0 $l \cdot min^{-1}$ und am stärksten bevorzugt von 1,0 bis 2,0 $l \cdot min^{-1}$.

In einer besonders bevorzugten Variante erfolgt das Zuführen des kosmetischen Feststoffs (F) und das Zuführen des Fluids (S) jeweils bei konstanter Strömungsgeschwindigkeit und jeweils bei einer Strömungsgeschwindigkeit von 0,25 bis 5,0 $l \cdot min^{-1}$, stärker bevorzugt von 0,50 bis 3,0 $l \cdot min^{-1}$ und am stärksten bevorzugt von 1,0 bis 2,0 $l \cdot min^{-1}$. Insbesondere bevorzugt sind beide Strömungsgeschwindigkeiten gleich.

Vorzugsweise beträgt das Gewichtsverhältnis zwischen dem kosmetischen Feststoffs (F), oder, falls der kosmetische Feststoff (F) in Schritt a) als Dispersion in einem Fluid bereitgestellt von dieser Dispersion, zu dem Fluid (S) in Schritt c) von 25:75 bis 75:25, stärker bevorzugt von 35:65 bis 65:35 und am stärksten bevorzugt von 45:55 bis 55:45.

Die Durchflussmenge kann beispielsweise mittels eines magnetisch-induktiven Durchflussmessgerätes bestimmt werden.

Das Mischverfahren gemäß der vorliegenden Erfindung wird üblicherweise als kontinuierliches Verfahren durchgeführt.

Das Fluid (S) und der kosmetische Feststoff (F) können, bevor sie dem statischen Mischer zugeführt werden, einander zugeführt werden. Üblicherweise werden die Stoffe hierbei mittels einer Zulaufgeometrie einander zugeführt. Beispielsweise werden die beiden Stoffe nicht einfach mittels eines simplen Y-, T-Rohres o.ä. einander zugeführt sondern eine Vorrichtung verwendet, die eine bestimmte räumliche Anordnung der einzelnen Komponenten vor der Zufuhr zu dem statischen Mischer (M) bewirkt. Vorzugsweise ist die Zulaufgeometrie in Schritt c) koaxial oder seitlich.

Bei einer seitlichen Zulaufgeometrie sind innerhalb der Zufuhrleitung, üblicherweise ein Rohr, Einbauten derart angeordnet, dass die zwei oder mehreren eintretenden Ströme in einen gemeinsamen Strom überführt werden, in dem die eintretenden Ströme nebeneinander angeordnet sind. Bei einer seitlichen Zulaufgeometrie entspricht üblicherweise die Zahl der nebeneinander angeordneten Ströme des gemeinsamen Stromes der Zahl der eintretenden Ströme. Es ist jedoch möglich, einen oder mehrere eintretende Ströme vor der Zusammenführung aufzuspalten und in dem gemeinsamen Strom abwechselnd anzuordnen.

Bei einer seitlichen Zulaufgeometrie sind innerhalb der Zufuhrleitung, üblicherweise ein Rohr, Einbauten derart angeordnet, dass die zwei oder mehreren eintretenden Ströme in einen gemeinsamen Strom überführt werden, in dem die eintretenden Ströme koaxial zueinander angeordnet sind. Bei einer koaxialen Zulaufgeometrie entspricht üblicherweise die Zahl der koaxial angeordneten Ströme des gemeinsamen Stromes der Zahl der eintretenden Ströme. Es ist jedoch möglich, einen oder mehrere eintretende Ströme vor der Zusammenführung aufzuspalten und in dem gemeinsamen Strom koaxial abwechselnd anzuordnen.

Da eine perfekte Zusammenführung ohne Verwirbelungen technisch in der Regel nicht möglich ist und/oder eine Mischung nach Zusammenführung aus Entropiegründen erfolgt, sind die Grenzflächen der einzelnen Ströme u.U. diffus.

Die Größe des Innenquerschnitts des Endes der Zulaufvorrichtung, welche dem statischen Mischer (M) zugewandt ist entspricht üblicherweise der Größe des Innenquerschnitt des statischen Mischers (M) ohne die darin befindlichen strömungsbeeinflussenden Elemente, welcher der Zulaufvorrichtung zugewandt ist. Insbesondere bevorzugt sind beide Innenquerschnitte nicht nur gleich groß sondern weisen auch die gleiche Form, beispielsweise kreisrund auf.

Fig. 2A und Fig 2B zeigen bevorzugte Vorrichtungen mit denen ein seitlicher bzw. koaxialer Zulauf des Fluids (S) und des kosmetischen Feststoffes (F) realisiert werden kann.

In dem Mischverfahren gemäß der vorliegenden Erfindung können eine oder mehrere weitere Komponenten, falls vorhanden, vorzugsweise kosmetische Komponenten bereitgestellt werden und dem statischen Mischer zugeführt werden. Falls vorhanden, beträgt die Anzahl dieser Komponenten üblicherweise nicht mehr als drei, vorzugsweise nicht mehr als eins. Diese können mit dem kosmetischen Feststoff (F) und/oder dem Fluid (S) vor der Zuführung in den statischen Mischer zugeführt werden, sowohl bevor das Fluid (S) und der kosmetische Feststoff (F) einander zugeführt werden als auch danach. Eine seitliche Zuführung zu dem statischen Mischer ist grundsätzlich möglich, jedoch nicht bevorzugt. Die gleichzeitige Zuführung einer oder mehrere Komponenten zueinander und/oder zu dem Fluid (S) und/oder

dem kosmetischen Feststoff (F) ist ebenfalls grundsätzlich möglich. Vorzugsweise besteht die Mischung in Schritt d) oder Schritt e), falls vorhanden, zu mindestens 75 Gew.%, stärker bevorzugt zu mindestens 85 Gew.%, noch stärker bevorzugt zu mindestens 95 Gew.%, insbesondere bevorzugt zu 100 Gew.% aus dem Fluid (S) und dem kosmetischen Feststoff (F).

Das Mischverfahren gemäß der vorliegenden Erfindung ist insbesondere geeignet große Materialmengen zu Mischen. Beispielsweise kann die Gesamtmenge der in dem Verfahren gemischten Materialien mindestens 250 kg betragen.

Vorzugsweise wird der Druck vor und nach dem statischen Mischer bestimmt, wobei das Verhältnis des Drucks vor dem statischen Mischer zu nach dem statischen Mischer während des gesamten Verfahrens zwischen 1,5 und 5,0 beträgt, stärker bevorzugt zwischen 2,0 und 4,0.

Das Produkt von Schritt d) weist vorzugsweise eine Mischgüte, bestimmt als Coefficient of Variation (CoV) von nicht mehr als 0,06, vorzugsweise von nicht mehr als 0,055 auf.

Außerdem Teil der vorliegenden Neuerung sind etwaige Kombinationen hier beschriebener Merkmale und/oder Abgrenzungen, insofern sie sich nicht gegenseitig ausschließen. Die Beschreibung der Neuerung betrifft bestimmte Ausführungsformen der Neuerung mit dem Ziel, diese zu veranschaulichen. Der zuständige Fachmann wird erkennen, dass weitere Modifikationen und Äquivalente der hier beschriebenen Ausführungsformen möglich sind. Solche Modifikationen und Äquivalente stellen auch einen Teil des Gesamtumfangs der beschriebenen Neuerung dar.

Ferner ist die vorliegende Erfindung durch die folgenden Punkte charakterisiert:

1. Ein Mischverfahren aufweisend die folgenden Schritte:

    a) Bereitstellen eines kosmetischen Feststoffs (F),
    b) Bereitstellen eines Fluids (S),
    c) Zuführen des kosmetischen Feststoffs (F) und des Fluids (S) in einen statischen Mischer (M), und
    d) Erhalten einer Mischung enthaltend den kosmetischen Feststoff (F) und das Fluid (S) aus dem statischen Mischer (M).

2. Das Mischverfahren gemäß Punkt 1, wobei das Fluid (S) ein Gel enthält, vorzugsweise ein kosmetisches Gel enthält, z.B. ein kosmetisches Hydrogel.

3. Das Mischverfahren gemäß Punkt 2, wobei das Gel, oder das bevorzugte kosmetische Gel, z.B. das kosmetische Hydrogel, ein nicht-newtonisches Gel ist.

4. Das Mischverfahren gemäß einem der vorangegangenen Punkte, wobei der kosmetische Feststoff partikelförmig ist.

5. Das Mischverfahren gemäß einem der vorangegangenen Punkte, wobei der kosmetische Feststoff (F) in Schritt a) in Form eines Fluids enthaltend den kosmetischen Feststoff (F) bereitgestellt wird.

6. Das Mischverfahren gemäß Punkt 5, wobei der Anteil des kosmetischen Feststoffes (F), bezogen auf das Gesamtgewicht des Fluids, inklusive des kosmetischen Feststoffes (F), 10,0 bis 50,0 Gew.% beträgt.

7. Das Mischverfahren gemäß einem der vorangegangenen Punkte 4 bis 6, wobei der partikelförmige kosmetische Feststoff Mikrokapseln enthält, vorzugsweise zu mindestens 75 Gew.%, stärker bevorzugt zu mindestens 90 Gew.% und am stärksten bevorzugt zu 100% aus Mikrokapseln besteht.

8. Das Mischverfahren gemäß Punkt 7, wobei die Mikrokapseln mindestens zwei Phasen aufweisen, vorzugsweise mindestens eine feste und mindestens eine fluide Phase aufweisen, wobei, vorzugsweise, die feste Phase Proteine, wie z.B. Gelatine; Celluloseether; synthetische und natürliche Polymere, insbesondere Polysaccharide, wie z.B. Alginate und Pektine, enthält.

9. Das Mischverfahren gemäß Punkt 8, wobei die Mikrokapseln Kern-Schale-Mikrokapseln sind.

10. Das Mischverfahren gemäß einem der vorangegangenen Punkte 4 bis 9, wobei der partikelförmige kosmetische Feststoff (F) oder die Mikrokapseln einen Feret-Durchmesser von 0,5 bis 20 mm aufweisen, vorzugsweise 1,0 bis 5,0 mm, mehr bevorzugt 1,4 bis 3,4 mm.

11. Das Mischverfahren gemäß einem der vorangegangenen Punkte, wobei das Verfahren ferner den folgenden

Schritt aufweist:

e) Abfüllen der Mischung erhalten aus Schritt d), vorzugsweise in ein oder mehrere kosmetische Packmittel, wie beispielsweise in einen oder mehrere Dispenser, Tiegel, Pumpspender, insbesondere Pumpspender, wie beispielsweise Schleppkolbenspender;

wobei, vorzugsweise, die mittlere Verweilzeit des kosmetischen Feststoffs (F) und des Fluids (S) zwischen dem Beginn von Schritt c) und dem Ende von Schritt e) nicht mehr als 45 Minuten beträgt.

12. Das Mischverfahren gemäß einem der vorangegangenen Punkte, wobei das Zuführen des kosmetischen Feststoffs (F) und/oder des Fluids (S) bei konstanter Strömungsgeschwindigkeit und/oder jeweils bei einer Strömungsgeschwindigkeit von 0,25 bis 5,0 $l \cdot min^{-1}$ erfolgt.

13. Das Mischverfahren gemäß einem der vorangegangenen Punkte, wobei der kosmetische Feststoff (F) in Form eines Fluids enthaltend den kosmetischen Feststoff (F) bereitgestellt wird, vorzugsweise als Dispersion in einem Fluid bereitgestellt wird, und die dynamische Viskosität $\eta$ des Fluids enthaltend den kosmetischen Feststoff (F) inklusive des kosmetischen Feststoffes (F), bestimmt bei einer Temperatur von 25°C, 1000 bis 6000 mPa·s, vorzugsweise 1500 bis 4500 mPa·s und am stärksten bevorzugt 1800 bis 2500 mPa·s beträgt.

14. Das Mischverfahren gemäß einem der vorangegangenen Punkte, wobei der kosmetische Feststoff (F) in Form eines Fluids enthaltend den kosmetischen Feststoff (F) bereitgestellt wird, vorzugsweise als Dispersion in einem Fluid bereitgestellt wird, und die Differenz zwischen der dynamischen Viskosität $\eta$ des Fluids enthaltend den kosmetischen Feststoff (F) inklusive des kosmetischen Feststoffes (F), bestimmt bei einer Temperatur von 25°C, und der dynamischen Viskosität $\eta$ des Fluids (S), bestimmt bei einer Temperatur von 25°C, zwischen -1000 und 1000 mPa·s, bevorzugt zwischen -500 und 500 mPa·s und am meisten bevorzugt zwischen -250 und 250 mPa·s.

15. Das Mischverfahren gemäß einem der vorangegangenen Punkte, wobei das Fluid (S) eine dynamische Viskosität $\eta$, bestimmt bei einer Temperatur von 25°C, von 1000 bis 6000 mPa·s, vorzugsweise 1500 bis 4500 mPa·s und am stärksten bevorzugt 1800 bis 2500 mPa·s aufweist.

16. Das Mischverfahren gemäß einem der vorangegangenen Punkte, wobei das Gewichtsverhältnis zwischen dem kosmetischen Feststoffs (F) oder, falls der kosmetische Feststoff (F) in Schritt a) in Form eines Fluids enthaltend den kosmetischen Feststoff (F) bereitgestellt wird, vorzugsweise als Dispersion in einem Fluid bereitgestellt wird, von diesem Fluid enthaltend den kosmetischen Feststoff (F) inklusive des kosmetischen Feststoffes (F), zu dem Fluid (S) in Schritt c) von 25:75 bis 75:25, vorzugsweise von 35:65 bis 65:35 und am stärksten bevorzugt von 45:55 bis 55:45 beträgt.

17. Das Mischverfahren gemäß einem der vorangegangenen Punkte, wobei die Zulaufgeometrie in Schritt c) koaxial oder seitlich ist.

18. Das Mischverfahren gemäß einem der vorangegangenen Punkte, wobei der statische Mischer strömungsbeeinflussende Elemente aufweist, die üblicherweise wendel-, steg-, lamellen-, gitterförmig oder als eine Mischung hiervon ausgelegt sind.

19. Das Mischverfahren gemäß einem der vorangegangenen Punkte, wobei das Verhältnis der Länge des statischen Mischers gemessen von dem Beginn bis zum Ende aller vorhandenen strömungsbeeinflussenden Elementen zu dem durchschnittlichen Innenquerschnitt des statischen Mischers entlang dieser Länge ohne die darin enthaltenen strömungsbeeinflussenden Elemente 1,0 bis 50 beträgt.

20. Das Mischverfahren gemäß einem der vorangegangenen Punkte, wobei der durchschnittliche Querschnitt des statischen Mischers gemessen von dem Beginn bis zum Ende aller vorhandenen strömungsbeeinflussenden Elemente zwischen 5,0 bis 25 $cm^2$ beträgt.

21. Das Mischverfahren gemäß einem der vorangegangenen Punkte, wobei die Länge des statischen Mischers gemessen von dem Beginn bis zum Ende aller vorhandenen strömungsbeeinflussenden Elemente 10 bis 120 cm beträgt.

22. Das Mischverfahren gemäß einem der vorangegangenen Punkte, wobei das Produkt von Schritt d) eine Mischgüte, bestimmt als Coefficient of Variation (CoV) von nicht mehr als 0,06, vorzugsweise von nicht mehr als 0.055 aufweist.

23. Produkt erhältlich aus dem Verfahren gemäß einem der vorangegangenen Ansprüche.

24. Verwendung eines statischen Mischers zum Mischen eines kosmetischen Feststoffs (F) und eines Fluids (S).

Experimenteller Teil:

Messmethoden:

Viskosität

Die Viskosität wird mittels einer Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 $min^{-1}$, verwendet.

Variationskoeffizient

Die Mischgüte wird anhand des Variationskoeffizienten (coefficient of variation oder kurz CoV) bestimmt. Für die Berechnung des Variationskoeffizienten CoV wird ein optisches Messverfahren gewählt. Das erzeugte Bildmaterial der Proben wird anhand einer Bildanalyse ausgewertet.

Zur Quantifizierung dieser wird ein optisches Messverfahren angewendet, das hier beschrieben wird. Für die Auswertung werden Gegenlichtaufnahmen der Materialproben erstellt. Dazu werden die einzelnen Proben vor einem LED-Panel platziert und mit einer *Canon Power Shot G9X Mark II* Kamera fotografiert. Sofern das äußere Gel transparent ist, nimmt mit ansteigendem Anteil von Mikrokapseln die Lichtdurchlässigkeit ab. Dieser Effekt spiegelt sich auch in den Intensitätswerten des aufgenommenen Bildmaterials wieder. Für diese Art der Auswertung werden Probenbehälter mit einer transparenten und ebenen Oberfläche und eine einheitliche Schichtdicke benötigt. Verwendet wurden quaderförmige Probenbehälter aus PMMA mit einem inneren Abmaß von 30 x 30 x 120 $mm^3$, die vollständig gefüllt wurden. Jede Probe wird grundsätzlich von zwei benachbarten Seiten fotografiert, da Inhomogenitäten innerhalb einer Probe nicht unbedingt durch die Betrachtung einer Seite erfasst werden können.

Die aufgenommenen Bilder wurden auf die Probe im Behälter mittels einer Bildbearbeitungssoftware (ImageJ) skaliert und zugeschnitten. In Bezug auf die hier aufgenommenen Fotos ergab sich ein Umrechnungsfaktor von 35,733 Pixel pro mm. Der zugeschnittene Bildausschnitt entsprach 27 x 99 $mm^2$. Anschließend wurden die Bildzuschnitte in Graustufen konvertiert und 3 x 3 $mm^2$ große Zellen (N) unterteilt.

Im nächsten Schritt werden die Mittelwerte $\overline{x}_{z_i}$ der Graustufenwerte jeder Zelle $z_i$, $i \in \{1, ..., N\}$ nach der Gleichung $\overline{x} = \frac{1}{N}\sum_{i=1}^{N} x_i$ berechnet, welche dann als Grauwert für die jeweilige Zelle angenommen werden. Ein erneutes Mitteln über alle $\overline{x}_{z_i}$ resultiert in einem mittleren Grauwert $\overline{x}$ über die gesamte Probe und beschreibt, wie viel Licht im Mittel durch jede einzelne Zelle dringt. Als Maß für die Homogenität einer Probe wird der Variationskoeffizient verwendet. Dieser wird mithilfe der Standardabweichung $\sigma = \sqrt{\frac{1}{N}\sum_{i=1}^{N}(x_i - \overline{x})^2}$ nach Gleichung $CoV = \frac{\sigma}{\overline{x}}$ für alle Proben berechnet. Ein Variationskoeffizient von CoV =0 beschreibt eine ideale Durchmischung, wohingegen die Komponenten bei CoV =1 komplett getrennt vorliegen.

Da es sich bei dem verwendeten Verfahren um ein indirektes Messverfahren handelt, werden die Ergebnisse relativ zu einigen Referenzmessungen ausgewertet. Hierzu wurden jeweils acht Proben von zwei Vorlagen vermessen und deren mittlere Grauwerte berechnet:

Vorlage 1: Fluid (S) ohne Mikrokapseln aus Schritt b) ($\overline{x}_{Vorlage1}$)
Vorlage 2: Mischung aus Mikrokapseln/Feststoff (F) und Fluid (S) aus Schritt a) ($\overline{x}_{Vorlage2}$)

Die beiden Referenzen aus den Vorlagen 1 und 2 erlauben eine obere und untere Grenze für die mittleren Grauwerte anzugeben. Zur Bestimmung dieser Grenzen wurde für jede dieser Proben der mittlere Grauwert berechnet und über alle Proben gemittelt, so dass sich die Mittelwerte $\overline{x}_{Vorlage1}$ und $\overline{x}_{Vorlage2}$ ergeben. Mithilfe dieser Grenzen

werden alle $\overline{x}$ und $\overline{x}_{z_i}$ wie folgt normiert:

$$\overline{x}_{normiert} = \frac{\overline{x} - \overline{x}_{Vorlage2}}{\overline{x}_{Vorlage1} - \overline{x}_{Vorlage2}}$$

Die Werte für CoV ergeben sich gemäß der vorstehenden Beschreibung. Als ideale Mischung bei einer 1:1 Mischung wird ein $\overline{x}_{normiert}$ von 0,254 bis 0,436 sowie ein Variationskeoffizient CoV im Bereich von 0 bis 0,0548 angenommen. Es wurden jeweils 4 Proben vermessen.

Schichtdicke der Kern-Schale Mikrokapseln und Feret-Durchmesser

Mindestens 20 Mikrokapseln werden isoliert in einer Petrischale bereitgestellt. Die Petrischale wird unter das Binokularmikroskop mit Messokular gestellt und für jede Mikrokapsel der größten ($d_l$) und kürzesten ($d_s$) messberare Durchmesser bestimmt. Anschließend wird für jede Mikrokapsel der mittlere Durchmesser $d_m$ bestimmt, welcher sich nach $d_m = (d_s+d_l)/2$ ergibt. Der Mittelwert aus $d_m$ aller Mikrokapseln gibt den gesuchten Feret-Durchmesser an. Die Standardabweichung für den ermittelten Feret-Durchmesser sollte nicht unter 0,25 mm liegen. Die Schichtdicke der Mikrokapseln wird ermittelt, indem sowohl der Kern $d_k$ als auch die Schale plus Kern ($d_m$) nach dem oben beschriebenen Verfahren vermessen wurden. $d_k$ ergibt sich indem der größte ($d_{kl}$) und kürzeste ($d_{ks}$) messberare Durchmesser des Kerns bestimmt wird und anschließend $d_k = (d_{ks}+d_{kl})/2$ für jede Mikrokapsel berechnet wird. Der Mittelwerte aller Mikrokapseln für $d_k$ und $d_m$ werden verwendet, um die Schichtdicke $S_k$ gemäß $S_k=d_m-d_k/2$ zu bestimmen.

Mittlere Bruchkraft des kosmetischen Feststoffes

Die Bruchkraft wurde mittels eines TA.XT plus Texture Analyser der Firma Stable Micro Systems an zehn einzelnen isolierten Mikrokapseln-gemessen und der Mittelwert bestimmt. Die Messung wurde mittels der Software Exponent derselben Herstellerfirma durchgeführt. Ein Stempel wird von einer Höhe oberhalb der Mikrokapseln mit einer Geschwindigkeit von 10 μm/s der Grundplatte entgegengefahren und währenddessen die aufgewendete Kraft gemessen. Von der ersten Berührung bis zum Bruch der Mikrokapsel steigt die gemessene Kraft. Der höchste Messwert bezeichnet die Bruchkraft.

Mittlere Verweilzeit

Die mittlere Verweilzeit wird durch Stoßmarkierung unter Verwendung eines farbigen Tracers ermittelt, der unmittelbar vor dem statischen Mischer in den Fluidstrom eingebracht wird.

Erfindungsgemäßes Beispiel:

Phase mit Gel ohne Mikrokapseln (Gel 1) umfasst die folgenden Komponenten:

| **Inhaltstoffe** | **Gel 1** |
|---|---|
| Aqua | 81,81% |
| Glycerin | 10,18% |
| Methyl propandiol | 3,99% |
| 1,2-Hexandiol | 1,00% |
| Phenoxyethanol | 0,80% |
| Locust Bean Gum | 0,30% |
| Xanthan Gum | 0,30% |
| Gellan Gum | 0,10% |
| PEG-40 Hydrogenated Castor Oil | 0,60% |
| Sodium Citrate | 0,40% |
| Sodium Hyaluronate | 0,45% |

(fortgesetzt)

| Inhaltstoffe | Gel 1 |
|---|---|
| Creatin | 0,06% |
| Kreatinin | 0,01% |

Die Mikrokapseln werden wie folgt hergestellt:

Zunächst wurde eine Ölphase und eine Alginatlösung bereitgestellt. Die nachfolgende Tabelle gibt die Anteile der Inhaltstoffe beider Lösungen an, wobei sich die Gew.-% Angaben auf das Gesamtgewicht beider Lösungen beziehen, so dass die Gewichtsverhältnisse beider Lösungen zueinander direkt berücksichtigt sind:

| Lösung | Inhaltstoffe | Anteil in Gew.-% |
|---|---|---|
| Ölphase | Octyldodecanol | 21,14 |
| | Dicaprylyl Carbonate | 10,56 |
| | Ethylhexyl Stearate | 10,56 |
| | Cetearyl Isoonanoate | 9,51 |
| | Simmondsia Chinensis Seed Oil | 0,81 |
| | Argania Spinosa Kernel Oil | 0,27 |
| | Tocopherol | 0,27 |
| | Perfume | 1,36 |
| | Mica | 0,04 |
| | CI 77491 | 0,01 |
| | CI 77891 | 0,06 |
| Alginatlösung | Aqua | 42,32 |
| | Sodium Alginate | 0,46 |
| | Methylpropanediol | 1,81 |
| | 1,2-Hexandiol | 0,46 |
| | Phenoxyethanol | 0,36 |

Zusätzlich wurde eine wässrige Calciumlösung enthaltend 2 Gew.-% Calciumchlorid, bezogen auf das Gesamtgewicht der Lösung bereitgestellt.

Die erfindungsgemäßen Partikel, bzw. Mikrokapseln wurde mit einem Spherisator M der Brace GmbH hergestellt. Das eingesetzte Gerät funktioniert über eine sogenannte Vibrationsdüsentechnik.

Zur Herstellung der Partikel, bzw. Mikrokapseln wurde die Ölphase durch die Innere- oder Kerndüse (0,6 mm Durchmesser) des Spherisators mit einem Druck von 135 mbar gedrückt. Die Alginatlösung wurde durch die äußere oder schalbildende Düse des Spherisators gedrückt, welche einen Durchmesser von 1,4 mm aufgewiesen hat. Der eingestellte Druck für die äußere Düse betrug 150 mbar. Die Vibrationsfrequenz des Spherisators wurde auf 75 Hz bei einer Amplitude für die Vibrationen von 1900 mV eingestellt. Die in diesem Verfahren hergestellten Tropfen, aufweisend einen Kern mit der Ölphase und eine äußere flüssige Hülle aus Alginatlösung, wurde in die Calciumchloridlösung getropft. Nach ca. 3 Minuten wurden die erhaltenen kugelförmigen Mikrokapseln mit einem geeigneten Sieb aus der Calciumchloridlösung entnommen und mit deionisiertem Wasser gewaschen. Nach dem Waschen wurden die erhaltenen Partikel, bzw. Mikrokapseln in eine Transportlösung gegeben. Das Gewichtsverhältnis der Partikel, bzw. Mikrokapseln, zur Transportlösung beträgt 70:30.

Die erhaltenen Partikel, bzw. Mikrokapseln weisen einen Feret-Durchmesser von 2,7 mm auf. Der mittlere Kerndurchmesser beträgt 2,3 mm. Die Hülle umfassend Calciumalginat und der Kern bildet sich aus den Inhaltstoffen der Ölphase.

Die erhaltenen Mikrokapseln enthalten eine Hülle umfassend Calciumalginat und einen Kern, welcher die Inhaltstoffe der Ölphase umfasst.

Die Transportlösung umfasst die folgenden Inhaltstoffe bezogen auf das Gesamtgewicht der Transportlösung:

| Lösung | Inhaltstoffe | Anteil in Gew.-% |
|---|---|---|
| Transportlösung | Aqua | 94 |
| | Methylpropanediol | 4 |
| | 1,2-Hexandiol | 1 |
| | Phenoxyethanol | 1 |

Die Mischung aus Transportlösung und Partikel, bzw. Mikrokapseln, wurde anschließend in der folgenden Gelzusammensetzung suspendiert (Anteil in Gew.-%):

| Inhaltsstoffe | Gel 2 |
|---|---|
| Aqua | 82,14% |
| Glycerin | 10,23% |
| Methyl propandiol | 4,01% |
| 1,2-Hexandiol | 1,00% |
| Phenoxyethanol | 0,80% |
| Locust Bean Gum | 0,30% |
| Xanthan Gum | 0,30% |
| Gellan Gum | 0,10% |
| PEG-40 Hydrogenated Castor Oil | 0,60% |
| Sodium Hyaluronate | 0,45% |
| Creatin | 0,06% |
| Kreatinin | 0,01% |

Da das Gel 2 keinen Komplexbildner aufweist, tritt keine Erweichung der Partikel, bzw. Mikrokapseln, auf. Folglich kommt es zu keiner Zerstörung der Partikel/Kapseln. Das Mischungsverhältnis von Gel 2 zu Transportlösung und Partikel, bzw. Mikrokapseln, beträgt 57,14 zu 42,86.
Die so erhaltene Mischung wird in einem Zulauf für den statischen Mischer zugegeben. Die Mischung mit Gel 1 erfolgte im statischen Mischer, wobei ein Verhältnis von 1:1 eingestellt wurde.
Es wurden die folgenden Viskositätswerte gemäß dem erfindungsgemäßen Messverfahren bestimmt:

| | |
|---|---|
| Gel 1: | 2200 mPas |
| Gel 2: | 2000 mPas |
| Gel 2 mitPerlenund Transportlösung: | 2150 mPas |
| Finales Produkterhalten nachderstatischenMischung: | 1900 mPas |

Versuchsaufbau:

Der Versuchsaufbau bestand aus den folgenden Komponenten.
Für die Förderung des schersensitiven Materials (Gel2 mit Perlen und Transportlösung) wird ein 50 L druckbeständiger Vorlagebehälter aus nichtrostendem Stahl verwendet. Der Behälter kann mit 0,5 bar relativem Druck beaufschlagt werden, womit über die Druckregelung Volumenströme zwischen 1 l/min und 15 l/min eingestellt werden können.
Der zweite Vorlagebehälter ist offen und fasst bis zu 80 L des gelartigen Füllguts (Gel1). Auf der Seite des druckbeständigen Vorlagebehälters ist bis zur Zusammenführung der Materialströme eine Rohrleitung mit der Nennweite DN 25 verbaut. Die gelartige Komponente auf der zweiten Seite wird durch ein Rohr mit der Nennweite DN 40 gefördert. Beide Ströme werden in ein DN 40er Rohr zusammengeführt, in dem auch der statische Mischer verbaut ist.
Der druckbeständige Vorlagebehälter ist für die Einstellung und die Überwachung des Drucks mit Druckregelventilen, einem Manometer und einem Überdruckventil ausgestattet.

In beiden Förderstrecken ist jeweils ein magnetisch-induktives Durchflussmessgerät verbaut um die Fluidströme überwachen zu können. Des Weiteren ist vor und nach dem statischen Mischer jeweils ein Drucksensor verbaut, um den Druckverlust über der Mischstrecke bestimmen zu können.

Als Pumpe wird eine Exzenterschneckenpumpe (Durchmesser Antriebswelle: 20 mm; Rotordurchmesser 15 mm) mit einem Rotor-Stator-System eingesetzt.

Die Zusammenführung der Materialströme erfolgt koaxial oderseitlich. Der Statische Mischer wurde in einem Mindestabstand von 20 cm hinter der Zusammenführung eingebaut.

Das Mischrohr weist je nach Länge des statischen Mischers eine Länge von 300 bis 1000 mm auf.

Es wurden die folgenden statischen Mischelemente verwendet:

| Mischelement | Kanalzahl | Kanalbreite | Länge je Element |
|---|---|---|---|
| SMX Mischer | 16 | 4 mm | 36,2 mm |
| SMXL Mischer | 8 | 8 mm | 134 mm |
| Kenics Mischer | 2 | 18 mm | 49 mm |

Die Anzahl der hintereinander angeordneten Mischer ist in der nachfolgenden Tabelle angeben:

| Versuchsbezeichnung | Anzahl Mischelemente | Länge Rohr in mm | Gesamtlänge Mischer in mm | L/D | Variationskoeffizienten (min... max) |
|---|---|---|---|---|---|
| Kenics 2 | 2 | 300 | 98 | 2,71 | 0,098 ... 0,167 |
| Kenics 4 | 4 | 300 | 196 | 5,41 | 0,052 ... 0,070 |
| Kenics 6 | 6 | 300 | 294 | 8,12 | 0,042 ... 0,066 |
| Kenics 8 | 8 | 660 | 392 | 10,83 | 0,051 ... 0,065 |
| Kenics 10 | 10 | 660 | 490 | 13,54 | 0,045 ... 0,064 |
| Kenics 12 | 12 | 660 | 588 | 16,24 | 0,049 ... 0,061 |
| Kenics 14 | 14 | 1000 | 686 | 18,95 | 0,049 ... 0,061 |
| SMXL 1 | 1 | 300 | 134 | 3,7 | 0,061 ... 0,102 |
| SMXL 2 | 2 | 300 | 268 | 7,4 | 0,050 ... 0,080 |
| SMXL 3 | 3 | 660 | 402 | 11,1 | 0,046 ... 0,063 |
| SMXL 4 | 4 | 660 | 536 | 14,81 | 0,047 ... 0,073 |
| SMXL 5 | 5 | 1000 | 670 | 18,51 | 0,048 ... 0,069 |
| SMXL 6 | 6 | 1000 | 804 | 22,21 | 0,053 ... 0,061 |
| SMX 4 | 8 | 300 | 144,8 | 4 | 0,045 ... 0,058 |

Hinter dem statischen Mischer erfolgte eine Abfüllung.

Die besten Ergebnisse werden bei einem Verhältnis der Volumenströme von 1:1 und einer Fließgeschwindigkeit von je 1,5 l/min erzielt. Bei einem statischen Mischer mit einem Durchmesser von DN 40 und einer Länge von etwa 60 cm wird innerhalb von 30 Sekunden ein ausreichender Durchmischungsgrad erreicht, so dass eine gleichmäßige Suspension der Mikrokapseln erfolgt ist.

<u>Vergleichsbeispiel mit dynamischen Mischer:</u>

Dazu wurden 1177 g des Gel 1 in einem IKA Magic Plants Mischer vorgelegt. Nach dem Einfüllen wurde der Mischer für 2 Minuten evakuiert. Anschließend wurde 98 g der Transportlösung ohne Mikrokapseln zugegeben. 225 g der hergestellten Mikrokapseln wurden von der Transportlösung getrennt und ebenfalls in den Mischer gegeben.

Es wurde mit dem Standardmischorgan von IKA 1,5 Minuten gemischt (20 U/min, 45 s Rechtslauf + 45 s Linkslauf). Das Produkt war homogen aber bereits sehr trüb, Perlen waren fast alle verformt oder aufgeplatzt. Das

Material wurde abgelassen und in ein Gefäß gefüllt. Der Mischversuch zeigte, dass ein dynamischer Mischer nicht geeignet ist, um die sensitiven Mikrokapseln zu mischen.

Ergebnisse:

Alle eingesetzten statischen Mischer zeigten eine zufriedenstellende Durchmischung der Produkte, wobei mit zunehmender Länge der Mischer ein höherer Durchmischungsgrad erreicht werden konnte. Insbesondere bei dem Einsatz der Kenics und SMXL Mischer kam es zu wenigen bis keinen Verformungen der MikrokKapseln beim Mischen. Es wurde kein Ausbluten der Kapseln ins umliegende Gel festgestellt.

## Patentansprüche

**1.** Ein Mischverfahren aufweisend die folgenden Schritte:

a) Bereitstellen eines kosmetischen Feststoffs (F),
b) Bereitstellen eines Fluids (S),
c) Zuführen des kosmetischen Feststoffs (F) und des Fluids (S) in einen statischen Mischer (M), und
d) Erhalten einer Mischung enthaltend den kosmetischen Feststoff (F) und das Fluid (S) aus dem statischen Mischer (M).

**2.** Das Mischverfahren gemäß Anspruch 1, wobei das Fluid (S) ein Gel enthält, vorzugsweise ein kosmetisches Gel enthält, z.B. ein kosmetisches Hydrogel, insbesondere bevorzugt ist das Gel, oder das bevorzugte kosmetische Gel, z.B. das kosmetische Hydrogel, ein nicht-newtonisches Gel.

**3.** Das Mischverfahren gemäß einem der vorangegangenen Ansprüche, wobei der kosmetische Feststoff partikelförmig ist.

**4.** Das Mischverfahren gemäß einem der vorangegangenen Ansprüche, wobei der kosmetische Feststoff (F) in Schritt a) in Form eines Fluids enthaltend den kosmetischen Feststoff (F) bereitgestellt wird, vorzugsweise beträgt der Anteil des kosmetischen Feststoffes (F), bezogen auf das Gesamtgewicht des Fluids, inklusive des kosmetischen Feststoffes (F), 10,0 bis 50,0 Gew.%.

**5.** Das Mischverfahren gemäß einem der vorangegangenen Ansprüche 3 oder 4, wobei der partikelförmige kosmetische Feststoff Mikrokapseln enthält, vorzugsweise zu mindestens 75 Gew.%, stärker bevorzugt zu mindestens 90 Gew.% und am stärksten bevorzugt zu 100% aus Mikrokapseln besteht.

**6.** Das Mischverfahren gemäß Anspruch 5, wobei die Mikrokapseln mindestens zwei Phasen aufweisen, vorzugsweise mindestens eine feste und mindestens eine fluide Phase aufweisen, wobei, vorzugsweise, die feste Phase Proteine, wie z.B. Gelatine; Celluloseether; synthetische und natürliche Polymere, insbesondere Polysaccharide, wie z.B. Alginate und Pektine, enthält, insbesondere bevorzugt sind die die Mikrokapseln Kern-Schale-Mikrokapseln.

**7.** Das Mischverfahren gemäß einem der vorangegangenen Ansprüche 3 bis 6, wobei der partikelförmige kosmetische Feststoff (F) oder die Mikrokapseln einen Feret-Durchmesser von 0,5 bis 20 mm aufweisen, vorzugsweise 1,0 bis 5,0 mm, mehr bevorzugt 1,4 bis 3,4 mm.

**8.** Das Mischverfahren gemäß einem der vorangegangenen Ansprüche, wobei das Verfahren ferner den folgenden Schritt aufweist:
e) Abfüllen der Mischung erhalten aus Schritt d), vorzugsweise in ein oder mehrere kosmetische Packmittel, wie beispielsweise in einen oder mehrere Dispenser, Tiegel, Pumpspender, insbesondere Pumpspender, wie beispielsweise Schleppkolbenspender;
wobei, vorzugsweise, die mittlere Verweilzeit des kosmetischen Feststoffs (F) und des Fluids (S) zwischen dem Beginn von Schritt c) und dem Ende von Schritt e) nicht mehr als 45 Minuten beträgt.

**9.** Das Mischverfahren gemäß einem der vorangegangenen Ansprüche, wobei

(i) das Zuführen des kosmetischen Feststoffs (F) und/oder des Fluids (S) bei konstanter Strömungsgeschwindigkeit und/oder jeweils bei einer Strömungsgeschwindigkeit von 0,25 bis 5,0 $l \cdot min^{-1}$ erfolgt; und/oder

(ii) der kosmetische Feststoff (F) in Form eines Fluids enthaltend den kosmetischen Feststoff (F) bereitgestellt wird, vorzugsweise als Dispersion in einem Fluid bereitgestellt wird, und die dynamische Viskosität $\eta$ des Fluids enthaltend den kosmetischen Feststoff (F) inklusive des kosmetischen Feststoffes (F), bestimmt bei einer Temperatur von 25°C, 1000 bis 6000 mPa·s, vorzugsweise 1500 bis 4500 mPa·s und am stärksten bevorzugt 1800 bis 2500 mPa·s beträgt; und/oder
(iii) der kosmetische Feststoff (F) in Form eines Fluids enthaltend den kosmetischen Feststoff (F) bereitgestellt wird, vorzugsweise als Dispersion in einem Fluid bereitgestellt wird, und die Differenz zwischen der dynamischen Viskosität $\eta$ des Fluids enthaltend den kosmetischen Feststoff (F) inklusive des kosmetischen Feststoffes (F), bestimmt bei einer Temperatur von 25°C, und der dynamischen Viskosität $\eta$ des Fluids (S), bestimmt bei einer Temperatur von 25°C, zwischen -1000 und 1000 mPa·s, bevorzugt zwischen -500 und 500 mPa·s und am meisten bevorzugt zwischen -250 und 250 mPa·s; und/oder
(iv) das Fluid (S) eine dynamische Viskosität $\eta$, bestimmt bei einer Temperatur von 25°C, von 1000 bis 6000 mPa·s, vorzugsweise 1500 bis 4500 mPa·s und am stärksten bevorzugt 1800 bis 2500 mPa·s aufweist; und/oder
(v) das Gewichtsverhältnis zwischen dem kosmetischen Feststoffs (F) oder, falls der kosmetische Feststoff (F) in Schritt a) in Form eines Fluids enthaltend den kosmetischen Feststoff (F) bereitgestellt wird, vorzugsweise als Dispersion in einem Fluid bereitgestellt wird, von diesem Fluid enthaltend den kosmetischen Feststoff (F) inklusive des kosmetischen Feststoffes (F), zu dem Fluid (S) in Schritt c) von 25:75 bis 75:25, vorzugsweise von 35:65 bis 65:35 und am stärksten bevorzugt von 45:55 bis 55:45 beträgt.

**10.** Das Mischverfahren gemäß einem der vorangegangenen Ansprüche, wobei

(i) die Zulaufgeometrie in Schritt c) koaxial oder seitlich ist; und/oder
(ii) der statische Mischer strömungsbeeinflussende Elemente aufweist, die üblicherweise wendel-, steg-, lamellen-, gitterförmig oder als eine Mischung hiervon ausgelegt sind; und/oder
(iii) das Verhältnis der Länge des statischen Mischers gemessen von dem Beginn bis zum Ende aller vorhandenen strömungsbeeinflussenden Elementen zu dem durchschnittlichen Innenquerschnitt des statischen Mischers entlang dieser Länge ohne die darin enthaltenen strömungsbeeinflussenden Elemente 1,0 bis 50 beträgt.

**11.** Das Mischverfahren gemäß einem der vorangegangenen Ansprüche, wobei der durchschnittliche Querschnitt des statischen Mischers gemessen von dem Beginn bis zum Ende aller vorhandenen strömungsbeeinflussenden Elemente zwischen 5,0 bis 25 $cm^2$ beträgt.

**12.** Das Mischverfahren gemäß einem der vorangegangenen Ansprüche, wobei die Länge des statischen Mischers gemessen von dem Beginn bis zum Ende aller vorhandenen strömungsbeeinflussenden Elemente 10 bis 120 cm beträgt.

**13.** Das Mischverfahren gemäß einem der vorangegangenen Ansprüche, wobei das Produkt von Schritt d) eine Mischgüte, bestimmt als Coefficient of Variation (CoV) von nicht mehr als 0,06, vorzugsweise von nicht mehr als 0.055 aufweist.

**14.** Produkt erhältlich aus dem Verfahren gemäß einem der vorangegangenen Ansprüche.

**15.** Verwendung eines statischen Mischers zum Mischen eines kosmetischen Feststoffs (F) und eines Fluids (S).

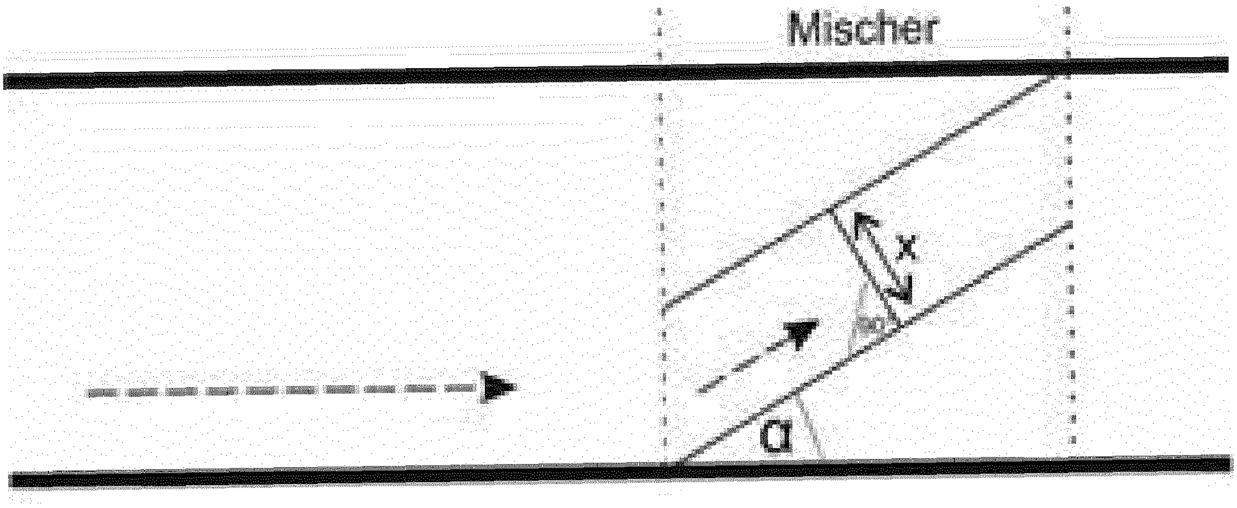


Fig. 1

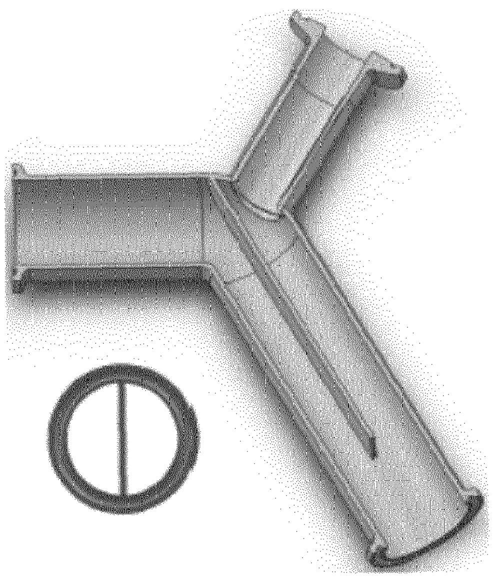

Fig. 2A

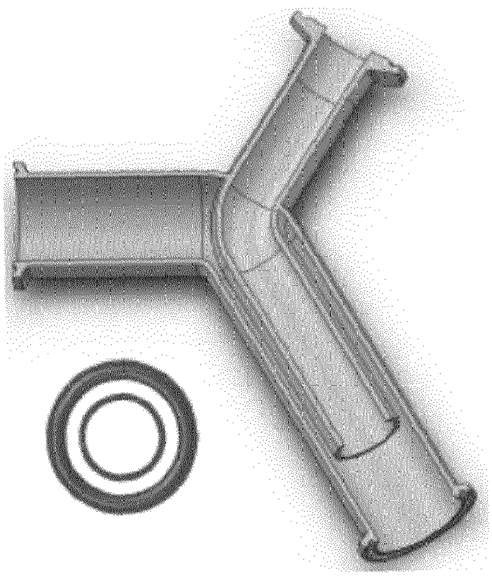

Fig. 2B

Europäisches Patentamt
European Patent Office
Office européen des brevets

# EUROPÄISCHER RECHERCHENBERICHT

Nummer der Anmeldung
EP 20 19 1588

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2015/374593 A1 (CETTI JONATHAN ROBERT [US] ET AL) 31. Dezember 2015 (2015-12-31)<br>* Absatz [0001] - Absatz [0003] *<br>* Absatz [0066] - Absatz [0082] *<br>* Absatz [0107] - Absatz [0115] *<br>* Absatz [0117] *<br>* Abbildung 1 *<br>----- | 1-15 | INV.<br>B01F5/06<br>A61K8/02<br>A61K8/11<br>A61K8/73<br>A61Q19/00 |
| X | DE 10 2007 057131 A1 (BEIERSDORF AG [DE]) 28. Mai 2009 (2009-05-28)<br>* Absatz [0001] *<br>* Absatz [0004] - Absatz [0013] *<br>* Absatz [0015] - Absatz [0018] *<br>* Absatz [0023] - Absatz [0039] *<br>* Abbildung 1 *<br>----- | 1-15 | |
| X<br><br>A | FR 2 984 287 A1 (OREAL [FR]) 21. Juni 2013 (2013-06-21)<br>* Seite 1, Zeile 1 - Zeile 3 *<br>* Seite 2, Zeile 11 - Zeile 31 *<br>* Seite 7, Zeile 13 - Zeile 17 *<br>* Seite 8, Zeile 28 - Seite 9, Zeile 4 *<br>* Seite 10, Zeile 1 - Zeile 6 *<br>* Seite 12 - Seite 15; Abbildung 22 *<br>* Seite 15, Zeile 11 - Zeile 23 *<br>* Abbildungen 1,3 *<br>----- | 1-4,7-15<br><br>5,6 | RECHERCHIERTE SACHGEBIETE (IPC)<br>B01F<br>A61K<br>A61Q |

Der vorliegende Recherchenbericht wurde für alle Patentansprüche erstellt

1

| Recherchenort | Abschlußdatum der Recherche | Prüfer |
|---|---|---|
| Den Haag | 3. Februar 2021 | Real Cabrera, Rafael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 20 19 1588

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-02-2021

| Im Recherchenbericht angeführtes Patentdokument | Datum der Veröffentlichung | Mitglied(er) der Patentfamilie | Datum der Veröffentlichung |
|---|---|---|---|
| US 2015374593 A1 | 31-12-2015 | CA 2950618 A1 | 07-01-2016 |
| | | CA 3099534 A1 | 07-01-2016 |
| | | EP 3160426 A1 | 03-05-2017 |
| | | MX 367383 B | 19-08-2019 |
| | | US 2015374593 A1 | 31-12-2015 |
| | | US 2017128333 A1 | 11-05-2017 |
| | | US 2017296449 A1 | 19-10-2017 |
| | | US 2019083374 A1 | 21-03-2019 |
| | | US 2020009032 A1 | 09-01-2020 |
| | | WO 2016003947 A1 | 07-01-2016 |
| DE 102007057131 A1 | 28-05-2009 | KEINE | |
| FR 2984287 A1 | 21-06-2013 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 9259030 B2